# EUROPEAN PATENT SPECIFICATION

(11) **EP 3 752 059 B1**
(45) Date of publication and mention of the grant of the patent: **02.04.2025**
(21) Application number: 19753851.5
(22) Date of filing: 08.02.2019
(51) Int. Cl.: A61B 5/05, A61B 5/00, A61B 5/055, A61B 5/103, A61B 5/11, A61B 6/00, A61B 5/22, G01R 33/48

(54) **METHODS, COMPUTER-READABLE MEDIA, MRI-COMPATIBLE TONGUE MEASUREMENT DEVICES, AND METHODS FOR TREATING DYSARTHRIA, SWALLOWING, AND MASTICATION DISORDERS ASSOCIATED WITH CNS OR PNS LESIONS**
VERFAHREN, COMPUTERLESBARE MEDIEN, MRT-KOMPATIBLE ZUNGENMESSVORRICHTUNGEN UND VERFAHREN ZUR BEHANDLUNG VON DYSARTHRIE, SCHLUCK- UND KAUSTÖRUNGEN IN ZUSAMMENHANG MIT ZNS-ODER PNS-LÄSIONEN
PROCÉDÉS, SUPPORTS LISIBLES PAR ORDINATEUR, DISPOSITIFS DE MESURE DE LANGUE COMPATIBLES AVEC L'IRM, ET PROCÉDÉS DE TRAITEMENT DE TROUBLES DYSARTHRITIQUES, DE DÉGLUTITION ET DE MASTICATION ASSOCIÉS À DES LÉSIONS DU SNC OU DU SNP

(30) Priority: 14.02.2018 US 201862630498 P
(43) Date of publication of application: 23.12.2020
(73) Proprietor: Baylor College of Medicine, Houston, TX 77030 (US)
(72) Inventor: PAPAGEORGIOU, Theodora Dorina, Houston, Texas 77006 (US); FROUDARAKIS, Emmanouil, Houston, Texas 77006 (US)
(74) Representative: Grünecker Patent- und Rechtsanwälte PartG mbB
(86) International application number: PCT/US2019/017186
(87) International publication number: WO 2019/160754

(56) References cited:
- WO-A2-2007/138598
- US-A1- 2011 263 968
- US-A1- 2013 118 504
- US-B1- 6 511 441
- HORWITZ ET AL.: "Fiber Optics Sensors Enable New MRI Applications", ECN MAGAZINE, 23 May 2014 (2014-05-23), XP055632595, Retrieved from the Internet <URL:https://www.ecnmag.com/article/2014/05/fiber-optic-sensors-enable-new-mri-applications> [retrieved on 20190418]
- ONO ET AL.: "Evaluation of tongue motor biomechanics during swallowing-From oral feeding models to quantitative sensing methods", JAPANESE DENTAL SCIENCE REVIEW, vol. 45, March 2009 (2009-03-01), pages 65 - 74, XP026447225, Retrieved from the Internet <URL:https://core.ac.uk/download/pdf/82425008.pdf> [retrieved on 20190418]
- HEBB ET AL.: "Visualizing Recovery of Cognitive Function in Stroke", JOURNAL OF BEHAVIORAL AND BRAIN SCIENCE, vol. 3, December 2013 (2013-12-01), pages 641 - 652, XP055632597, Retrieved from the Internet <URL:https://file.scirp.org/pdf/JBBS_2013123114320583.pdf> [retrieved on 20180410]
- MALANDRAKI ET AL.: "Functional Magnetic Resonance Imaging of Swallowing Function: From Neurophysiology to Neuroplasticity", HEAD NECK, vol. 33, no. 01, September 2011 (2011-09-01), pages S14 - S 20, XP055632602, Retrieved from the Internet <URL:https://www.ncbi.nlm.nih.gov/pmclarticles/PMC3747973> [retrieved on 20190409]
- SOROS ET AL.: "fMRI-Compatible Registration of Jaw Movements Using a Fiber-Optic Bend Sensor", FRONTIERS IN HUMAN NEUROSCIENCE, vol. 4, no. 24, 22 March 2010 (2010-03-22), pages 1 - 8, XP055632607, Retrieved from the Internet <URL:http://www.readcube.com/articles/10.3389/fnhum.2010.00024> [retrieved on 20190418]

## Description

### BACKGROUND OF THE INVENTION

The lower cranial nerves control pharyngeal and laryngeal function (swallowing and mastication), and tongue movement. Glossopharyngeal (Gp/CNIX) and hypoglossal (Hg/CNXII) nerve damage can result in a complete or partial weakness or paralysis of the areas served by these nerves and, in turn, widespread motor and somatosensory reorganization. The phenotypic presentation of Hp/CNXII and Gp/CNIX supranuclear (central nervous system) or infranuclear (peripheral nervous system) lesions in humans results in tongue movement, swallowing, mastication, and speech articulation difficulties.

WO 2007/138598 A2 discloses a method of rehabilitation management, comprising: providing a plurality of patients fitted with sensors; assigning tasks to said patients by a therapist; rehabilitating said patients not under the direct attention of said therapist; and monitoring the rehabilitating using data acquired by the sensors during performance of the tasks.

US 2011/263968 A1 relates to techniques and systems for training a subject to modify his or her own neuronal activity within a selected brain region.

US 2013/118504 A1 discloses methods, devices, and systems controlled delivery of a therapy, such as a stimulant, to a mouth of a subject via an oral device positioned in a secured configuration in the mouth.

### BRIEF SUMMARY OF THE INVENTION

The invention is set out in the appended set of claims. Any "embodiment" or "example" which is disclosed in the description but not covered by the claims should be considered as presented for illustrative purpose only.

The invention can be summarized by the appended claims.

### BRIEF DESCRIPTION OF THE DRAWINGS

For a fuller understanding of the nature and desired objects of the present invention, reference is made to the following detailed description taken in conjunction with the accompanying drawing figures wherein like reference characters denote corresponding parts throughout the several views.

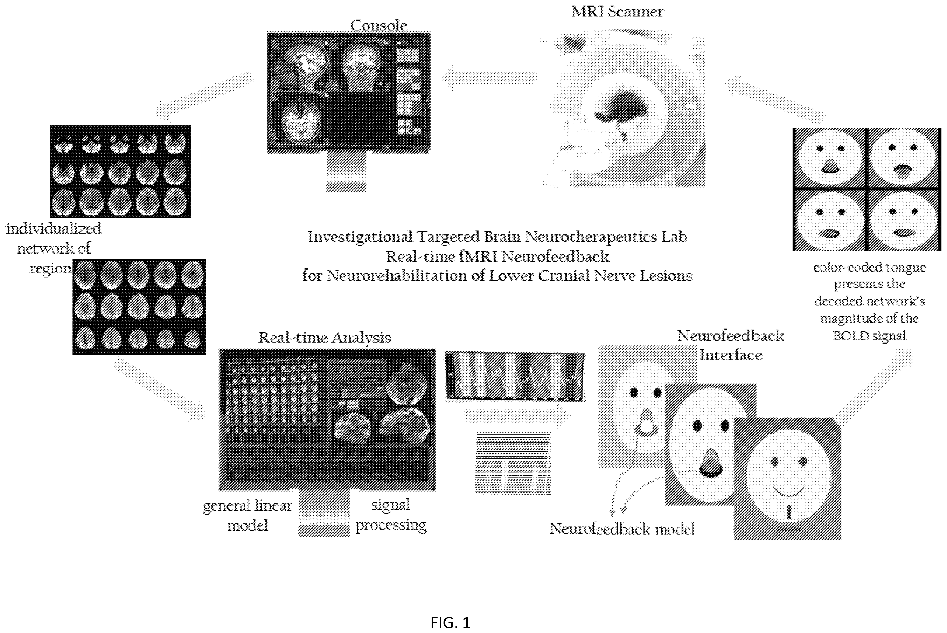

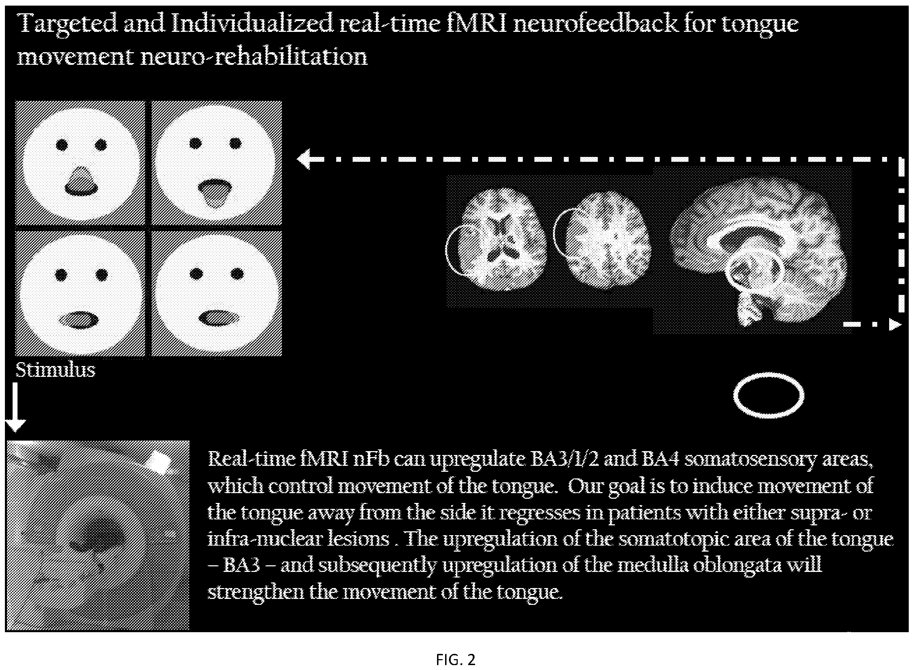

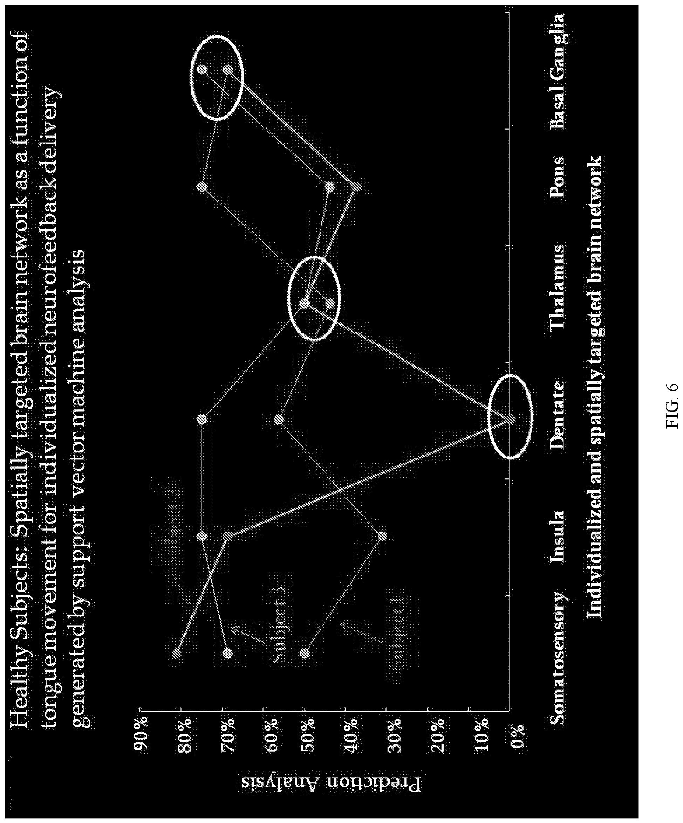

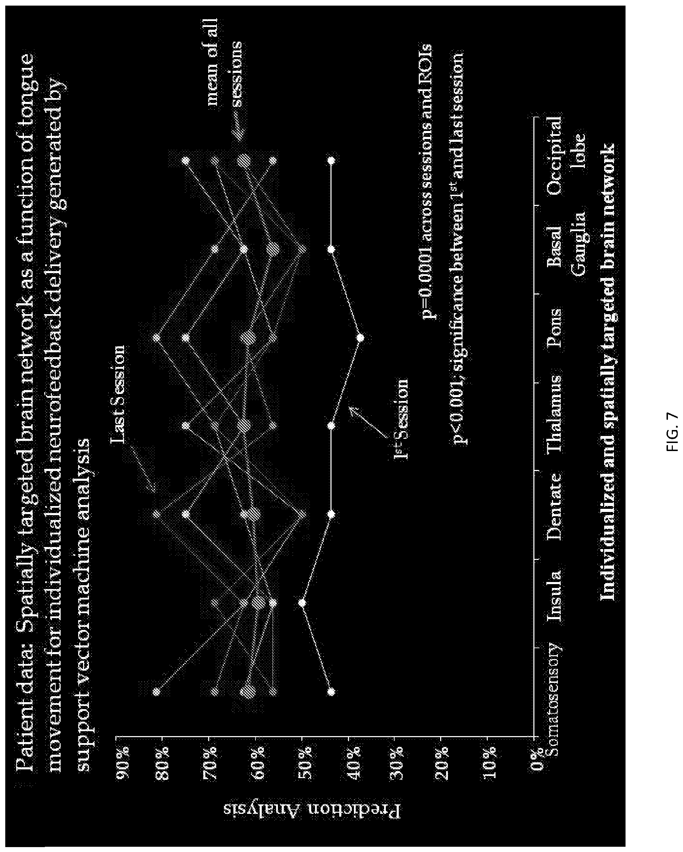

FIG. 1 depicts a schematic view of an example of the method disclosed in the context of an Rt-fMRI nFb paradigm for tongue motor movement neuro-rehabilitation following supra- or infra-nuclear injury to the regions of interest that control the movement of the tongue. The targeted and individualized approach is a closed-loop system including: (i) signal acquisition via the MRI scanner; (ii) signal processing/analysis decoded in real-time, and (iii) neurofeedback, which delivers the mean hemodynamic signal intensity of a patient's targeted and individualized area during performance of the task in real-time; this means the hemodynamic signal intensity is dynamically computed, meaning it is specific for each tongue movement and updated during the scan to incorporate the patient's improvement in upregulating the areas that regulate tongue movement. The patient is presented with four conditions and requested to train on the following visual task, while s/he is laying supine inside the MRI and receiving neurofeedback: (i) upward movement of the tongue (touching the hard palate); (ii) downward movement of the tongue (touching the soft palate); (iii) rightward movement of the tongue (touching the right cheek along the buccinator and masseter muscles); (iv) leftward movement of the tongue (touching the left cheek along the buccinator and masseter muscles). Each of these conditions is interleaved with a rest condition. The goal of the neurofeedback is to achieve maximal upregulation (100% increase of the BOLD signal) of the targeted area in comparison to baseline levels. The neurofeedback interface is presented with the following magnitudes of fill-in colors of the tongue (FIG. 2), which represent the magnitude of the BOLD signal of the targeted and individualized areas (FIG. 6 and FIG. 7), which control the movement of the tongue and where neurofeedback is delivered from: (i) -100%, complete downregulation of the targeted area; (ii) -75% downregulation of the targeted area; (iii) -50% downregulation of the targeted area; (iv) -25% downregulation of the targeted area; (v) 0% baseline levels of the targeted area; (vi) 25% upregulation of the targeted area; (vii) 50% upregulation of the targeted area; (viii) 75% upregulation of the targeted area; (xi) 100% upregulation of the targeted area. In this example, the targeted ROI is Brodmann area 3 (circled). The mean BOLD signal in Brodmann area 3 is estimated in real time and presented to the patient via the magnitude of the filled-in color of the tongue; i.e., the degree of red fill-in indicates the upregulation of the BOLD signal intensity of Brodmann area 3 (FIG. 1) or other network areas (FIG. 6 and FIG. 7). When the BOLD signal intensity of Brodmann area 3 (FIG. 1) or other network areas (FIG. 6 and FIG. 7) become downregulated, the fill-in color of the tongue becomes white. Baseline levels of the BOLD signal intensity of the targeted areas is represented by pink color. To summarize: (i) upregulation of the tongue is presented with red fill-in color of the tongue; (ii) downregulation of the tongue is presented with white fill-in color of the tongue; (iii) baseline levels, referred to as resting state of the tongue is presented with pink color of the tongue. Thus, the patient uses the fill-in of the dot (neurofeedback interface which denotes the BOLD signal of Brodmann area 3) to determine how successful his/her strategy is and modulate accordingly.
FIG. 2 depicts the neurofeedback stimulus which reflects the magnitude of BOLD signal, and in turn neuronal activation.
FIG. 3 depicts the configuration of the functional magnetic resonance imaging (fMRI)-compatible tongue sensor.

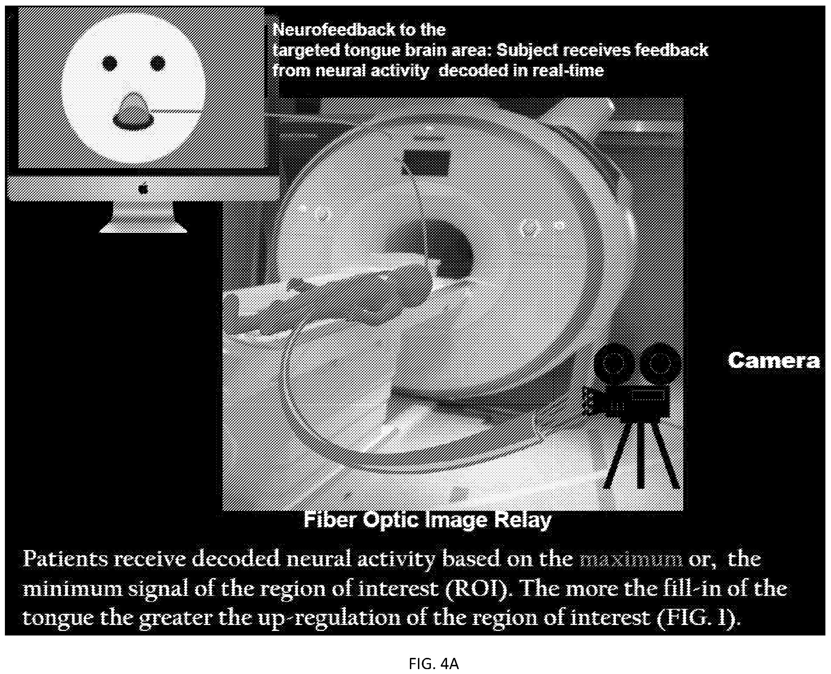

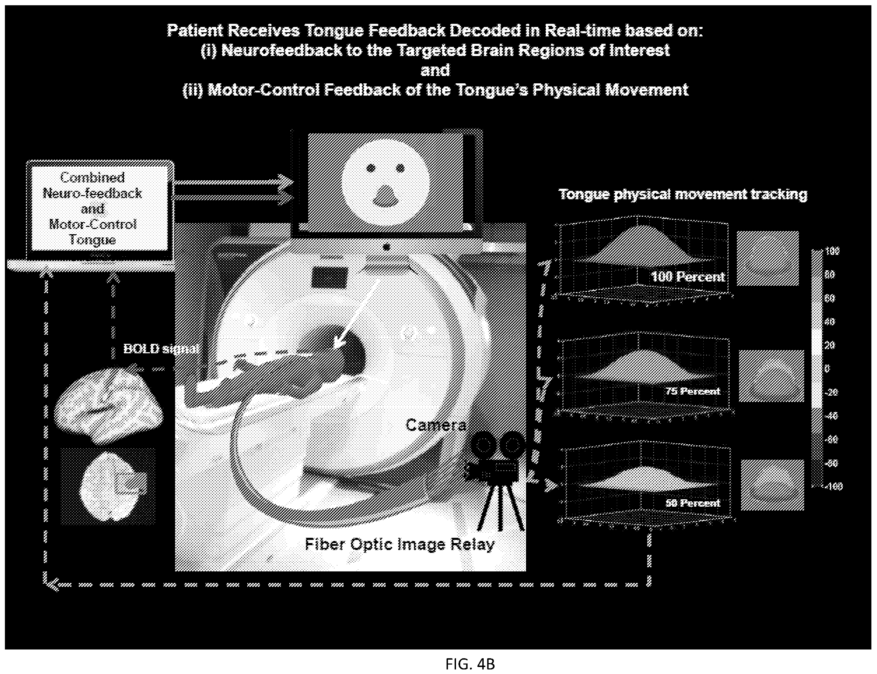

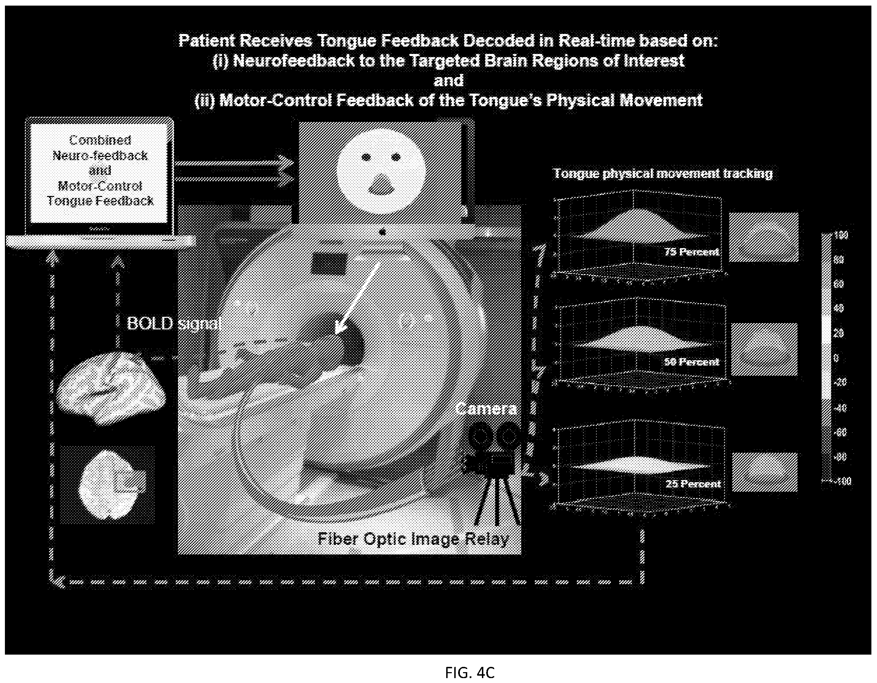

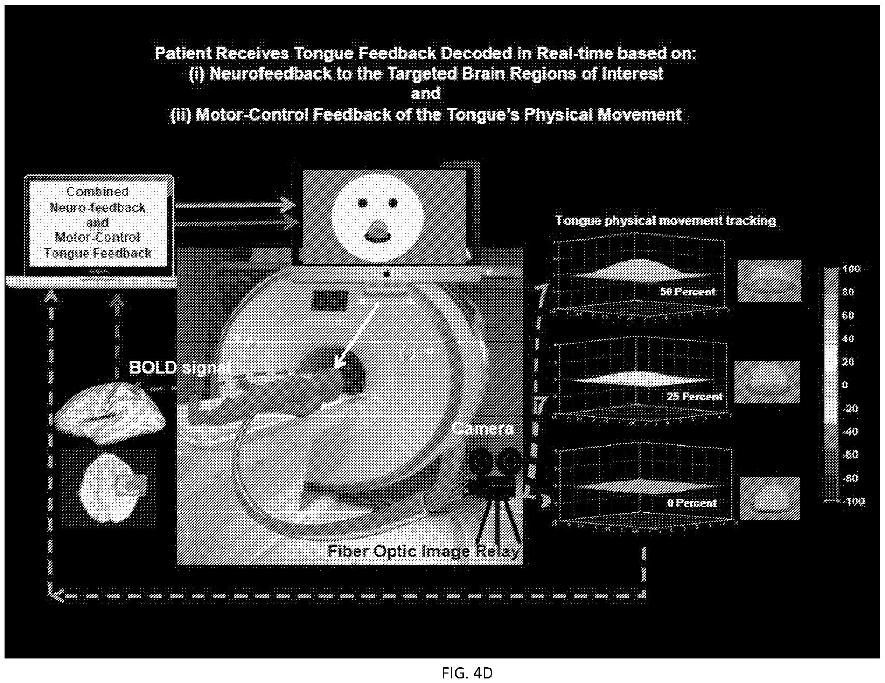

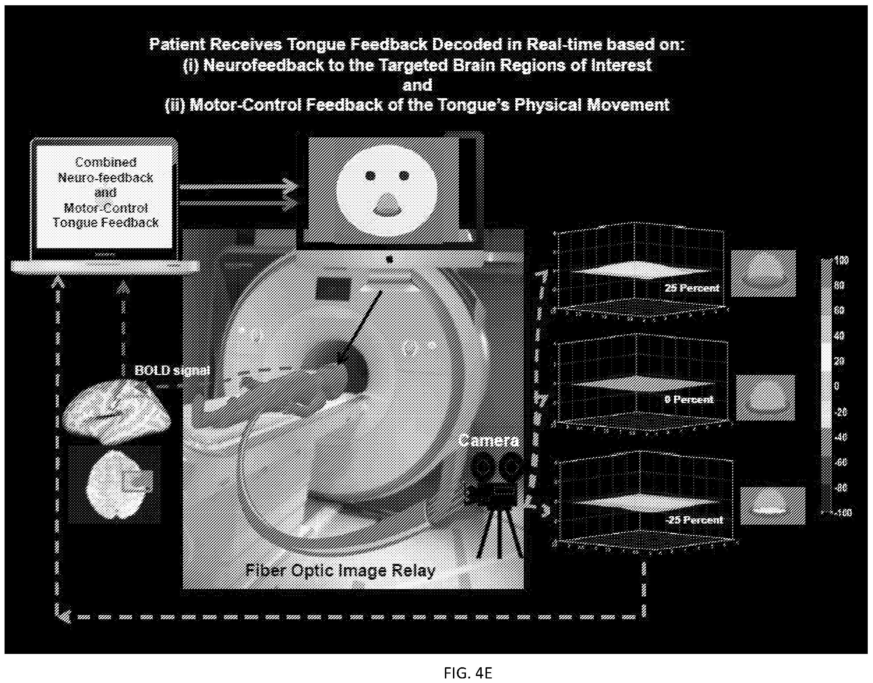

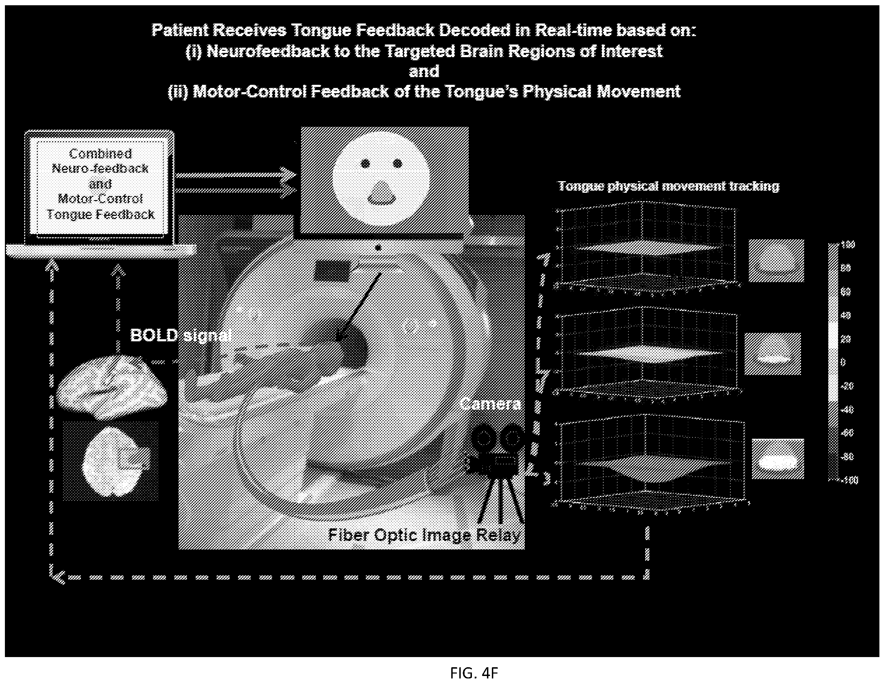

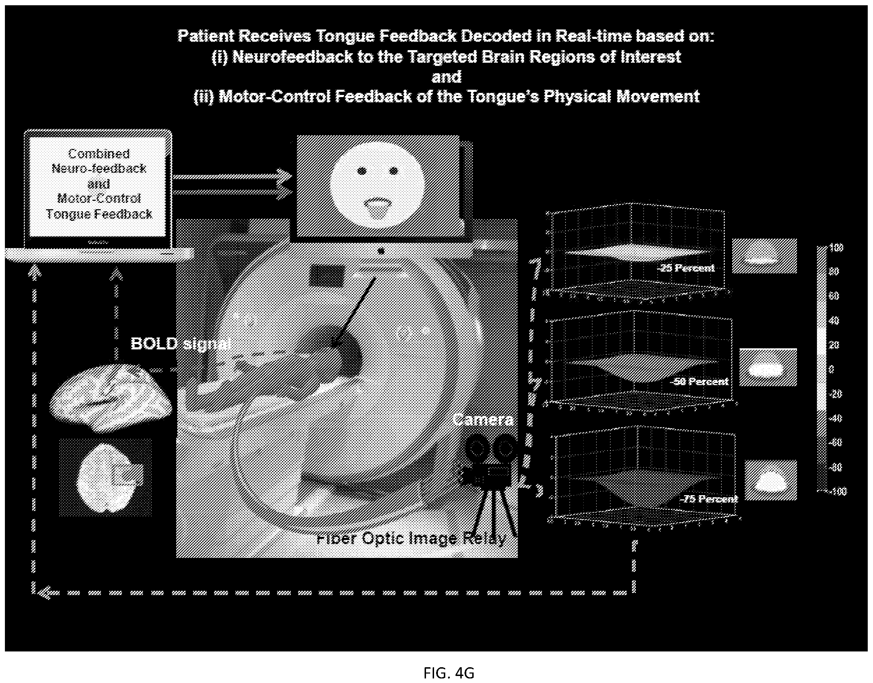

FIGS. 4A-G are a series of images in cartoon form depicting an example of the method.
FIG. 5A is an fMRI-generated image of the tongue of a healthy subject with normal hypoglossal interactions.
FIG. 5B is an fMRI-generated image that suggests the presence of muscle wasting with fat replacement of the anterior third of the tongue.
FIG. 6 depicts spatially targeted brain networks as a function of tongue movement for individualized neurofeedback delivery generated by support vector machine analysis for three healthy subjects.
FIG. 7 depicts a spatially targeted brain network as a function of tongue movement in a patient for individualized neurofeedback delivery generated by support vector machine analysis over a plurality of sessions.
FIG. 8 depicts a series of fMRI images comparing up and down, left and right tongue movement.
FIG. 9A depicts an fMRI image of left-side tongue deviation on a clinical exam evidenced by bilateral atrophy of the anterior third of the tongue but more so of the left side (arrows) on T2 flair. T2 hyper-intensities (validated on T1) show a greater extent of the left anterior third of the tongue muscle replaced with fat.
FIGS. 9B and 9C depict a general linear model showing significant (p=0.001) BOLD signal modulation after eleven rt-fMRI neurofeedback sessions. FIG. 9B depicts the patient's brain after the first session. FIG. 9C depicts the patient's brain after the eleventh session.
FIG. 10 depicts a box diagram of an embodiment of the system of the invention.
FIGS. 11A(i)-11A(iii) depict schematics of the design of embodiments of fMRI compatible tongue sensors. The designs are built on the principle that the reflected light emitted from the optic fiber will depend on the distance of an object to the optic fiber ending that can be detected by a photodetector. Different tongue movements will lead to different signals in the four channels of the optic fiber as illustrated in FIG. 11B.
FIG. 12, Panels A-C depict a working development setup for the MRI compatible tongue measurement device. FIG. 12, Panel A depicts the light to voltage sensor (TSL250R, AMS), coupler of the illumination (black fiber) and detection paths (white fiber) with an optic fiber coupler (FCMM50-90A, Thorlabs) and the IR source (M850L2, Thorlabs). FIG. 12, Panel B depicts coupling of the optic fiber to the photodetector with UV curing adhesive. FIG. 12, Panel C depicts voltage traces after an object is placed at the end of the optic fiber.

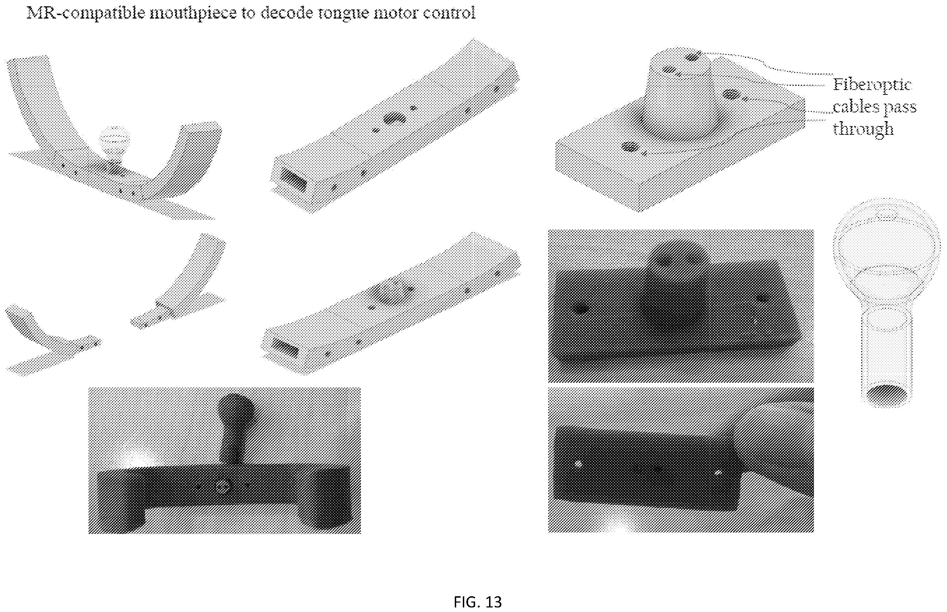

FIG. 13 depicts a magnetic resonance compatible mouthpiece according to various embodiments of the invention.

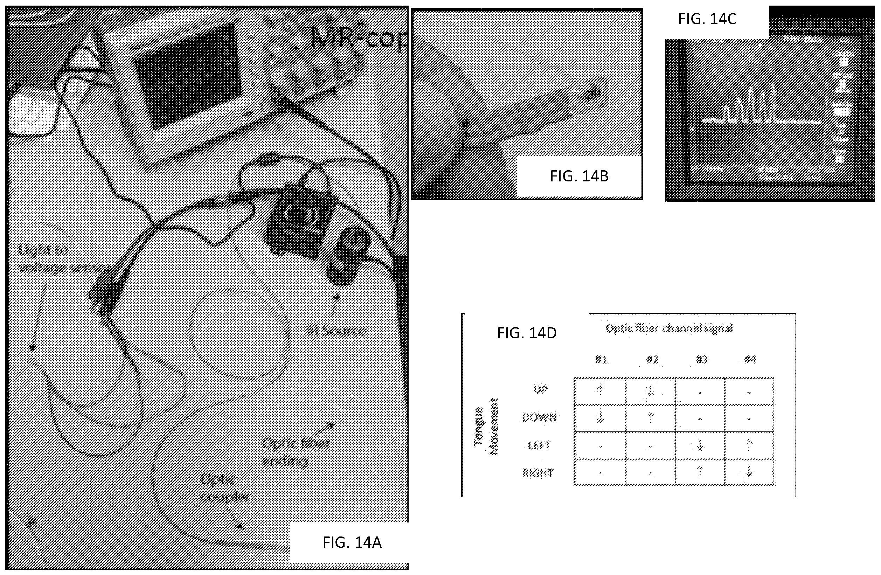

FIG. 14A depicts a light to voltage sensor (TSL250R, AMS), coupler of the illumination (black fiber) and detection paths (white fiber) with an optic fiber couples (FCMM50-90A, Thorlabs) and the infrared (IR) source (M850L2, Thorlabs).
FIG. 14B depicts coupling of the optic fiber to the photodetector with ultraviolet (UV)-curing adhesive.
FIG. 14C depicts voltage traces of a tongue acquired by placing the tongue at the end of the optic fiber.
FIG. 14D depicts how this MR-compatible sensor is built on the principle that the reflected light emitted from the optic fiber will depend on the distance of an object to the optic fiber ending which can be detected by a photodetector. Tongue movements generated the above signals in the four channels of the optic fiber: (i) upwards direction produced increased signal in channel 1; (ii) downwards movement increased signals in channel 2; (iii) right movement increased signal in channel 3; and (iv) left movement increased signal in channel 4.

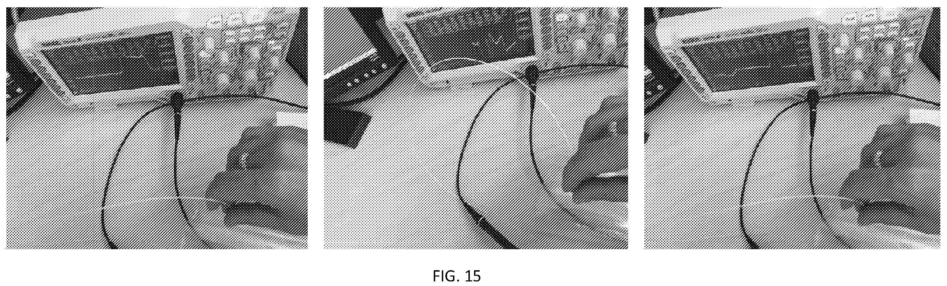

FIG. 15 depicts an example of a method of the invention.
FIG. 16 shows the BOLD signal's neuromodulation during swallowing when we compared the first session/scan (negative beta coefficient computed by general linear modeling) with the seventh session/scan (positive beta coefficient computed by general linear modeling) of a patient with neonatal hypoglassal and glossopharyngeal injury. GLM-generated BOLD spatial patterns show that neurofeedback engages insula, middle, inferior, and medial frontal gyrus areas which control proprioceptive awareness and attention to achieve a voluntary, consistent, and controlled swallowing rate, force, and velocity.

### DEFINITIONS

The instant invention is most clearly understood with reference to the following definitions.

As used herein, the singular form "a," "an," and "the" include plural references unless the context clearly dictates otherwise.

Unless specifically stated or obvious from context, as used herein, the term "about" is understood as within a range of normal tolerance in the art, for example within 2 standard deviations of the mean. "About" can be understood as within 10%, 9%, 8%, 7%, 6%, 5%, 4%, 3%, 2%, 1%, 0.5%, 0.1%, 0.05%, or 0.01% of the stated value. Unless otherwise clear from context, all numerical values provided herein are modified by the term about.

As used in the specification and claims, the terms "comprises," "comprising," "containing," "having," and the like can have the meaning ascribed to them in U.S. patent law and can mean "includes," "including," and the like.

Unless specifically stated or obvious from context, the term "or," as used herein, is understood to be inclusive.

As used herein, the term "blood-oxygen level dependent" or "BOLD" means the ratio of oxygenated to de-oxygenated hemoglobin and reflects the neuronal activity.

As used herein, the term "neurofeedback" includes training subjects to neuro-modulate the magnitude and spatial extent of the BOLD signal in targeted brain regions of interest (ROIs) by displaying the BOLD signal to a subject in real-time as nFb with the goal to enhance a particular behavior (FIGS. 1 and 2). The method for targeted and individualized rt-fMRI nFb training disclosed herein specifically aims to accomplish one or more of the following: (i) it strengthens pathways that bypass the ROI of cortical damage (up- or down-stream in reference to the lesion); (ii) its induction of reorganizing functional sensory and motor pathways is direct and targeted compared to what behavioral strategies (speech exercises) provide; and (iii) it can bypass the lesion, as it can capitalize on the redundancy of functionally associated and intact pathways to the lesioned one; and (iv) the training via individualized and targeted neurofeedback strengthens: (a) pathways that can bypass the lesion and/or (b) intact islands of neurons that reside within the lesion.

As used herein, the term "machine learning" means computational methods and models that can learn from each patient's data, which reflects the effects of the pathology/lesion on the network and make data-driven decisions to predict future data. These computational techniques allow effective training of patients for neuro-rehabilitation purposes because they focus on the specific pathology of the patient.

Ranges provided herein are understood to be shorthand for all of the values within the range. For example, a range of 1 to 50 is understood to include any number, combination of numbers, or sub-range from the group consisting 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 (as well as fractions thereof unless the context clearly dictates otherwise).

### DETAILED DESCRIPTION OF THE INVENTION

Various aspects and embodiments of the invention treat disorders associated with impaired control of the patient's tongue due to nerve damage by identifying the regions of the patient's brain that are involved in movement of the tongue, including the damaged brain region, and then applying neurofeedback techniques to induce plasticity while monitoring the motion of the patient's tongue. The neurofeedback modulates or neuromodulates patterns of activation in the patient's brain to assist the patient in learning to utilize intact regions of the brain to control the tongue, thereby improving the patient's control over the tongue, improving motor behavior and thereby treating the disorder. In other aspects, the invention provides software and a system that support the practice of the method. In order to facilitate monitoring of the movement of the tongue during the real-time fMRI neurofeedback, the invention provides an MRI-compatible tongue sensor and measurement device.

### Method of treating a swallowing, mastication, or speech articulation disorder

Disclosed is a method of treating a swallowing, mastication, or speech articulation disorder associated with a lesioned brain region or with a lesioned tongue in a patient, by: measuring a magnitude and a spatial extent of activation in a plurality of brain regions of the patient during movement of the tongue by functional magnetic resonance imaging (fMRI), generating a map of an individualized network of brain regions that control tongue movement, identifying at least one intact brain region functionally associated with the lesioned brain region based on the map of the individualized network of brain regions, tracking at least one tongue movement of the patient; providing neuronal feedback to the patient based on a level of activation of the at least one intact brain region identified and the at least one tongue movement; and instructing the patient to change at least one motor behavior of the tongue based on the neuronal feedback, thereby treating the disorder.

The disorder may be any wherein the patient's control over the movement of their tongue is impaired. The lesioned brain region may be any portion of the brain involved in the movement of the tongue. Alternatively, the lesioned tongue region may be any portion of the tongue. The method may be effectively performed on a wide variety of patients, therefore the nature and extent of the damage are not particularly limited. In various embodiments, the patient has a nerve injury. In various embodiments, the swallowing, mastication, or speech articulation disorder is caused by glossopharyngeal or hypoglossal nerve damage. In various embodiments, the nerve damage is at the supranuclear level in reference to where the glossopharyngeal or hypoglossal nerves originate. A supranuclear lesion is upstream of the medulla oblongata of the brainstem, and thus it refers to cortical and subcortical brain areas. In various embodiments, the nerve damage is at the infranuclear level of the glossopharyngeal or hypoglossal nerves. An infracuclear lesion is downstream of the medulla oblongata of the brainstem, and thus it refers to tongue lesioned areas mostly.

The magnitude and spatial extent of activation may be measured by detecting a blood-oxygen level dependent (BOLD) signal. Without wishing to be limited by theory, different patients will have different brain regions involved in control of the tongue and each individual's brain will have regions with a different spatial extent. FIGS. 6, 7 and 8 illustrate activity in different brain regions in different patients as well as different spatial extents for the regions. Accordingly, the real-time fMRI neurofeedback method can record measurements of activity of a plurality of regions in the patient's brain during movement of the tongue. In various embodiments, the entire brain of the patient, i.e. all brain regions, is measured.

Based on the measurements of the patient's brain during movement of the tongue, a map of an individualized network of brain regions that control tongue movement is generated. The map is individualized because, as discussed above, different brain regions are involved in movement of the tongue in different patients and the spatial extent of the brain regions is different from patient to patient. In various embodiments, generating the map of an individualized network of brain regions further comprises decoding each tongue movement (upwards, downwards, leftwards, rightwards) by correlating (e.g., by general linear modelling) the level of BOLD signal during the specific movement of the tongue recorded by an MRI-compatible tongue sensor or, by classifying the level of the BOLD signal during the specific movement of the tongue recorded by an MRI-compatible tongue sensor using machine learning and deep learning computational methods.

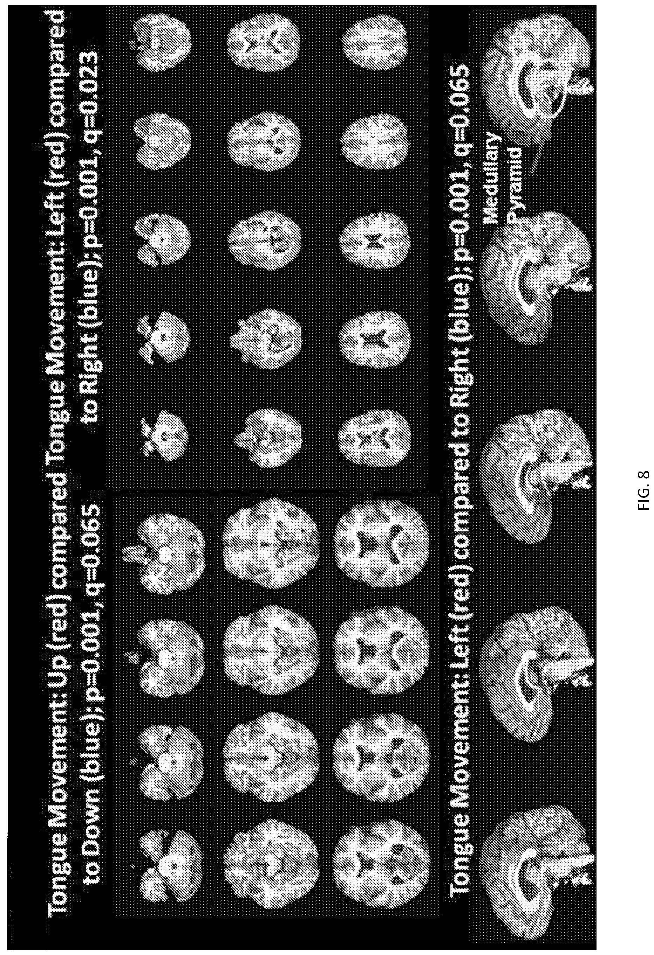

Accordingly, the map will be different from patient to patient. The map takes into account both the regions that are activated during tongue movement and the area of the brain that the region occupies. In various embodiments, the map may be generated using machine learning, such as support vector machine (SVM), or deep learning, such as neural networks methods. After a general linear model (GLM) (Fig. 8) is applied to decode the movement of the tongue in the brain then train/test (with "train" in machine learning referring to classifying and generating a model) and test in machine learning means to make predictions) is performed and test/train on the regions of interest generated to predict their validity and the reliability of their activation over several fMRI (functional localizer) runs that one session is composed of and across fMRI sessions (FIG. 6 and FIG. 7).

Once the individualized map is generated, at least one intact region of the brain that is functionally associated with the lesioned region is identified. The intact region is "functionally associated" with the lesioned region, meaning that the intact region is involved in the movement of the tongue and may fulfill a similar role to the lesioned region. In various embodiments, the at least one intact brain region includes Brodmann Area 3A, Brodmann Area 3B, Brodmann Area 2/1, Brodmann Area 4, the cerebellum/dentate, insula, basal ganglia, and/or thalamus. In various embodiments, the at least one intact brain region includes Brodmann Area 6, Brodmann Area 9, primary motor area, precentral gyrus, middle frontal gyrus, medial frontal gyrus, inferior frontal gyrus.

Training of the patient for tongue neuro-rehabilitation can include neurofeedback delivered to the patient while monitoring the activation in the individualized network of brain regions and tracking the movement of the tongue. The movement of the tongue may be tracked using an MRI-compatible tongue sensor as described herein. In various embodiments, the neuronal feedback is delivered from the intact region of the brain functionally associated with the lesioned region. In various embodiments, during real-time fMRI neurofeedback, the patient endeavors to fill in completely the tongue color with red (this color or any other color one can chose to denotes the upregulation or increase in BOLD signal from the intact region functionally associated with the lesioned region) via maximal degree of movement of the tongue in of up, down, left, and/or right direction. This maximal degree of movement of the tongue in any of these directions has to be sustainable and consistent. This is achieved by placing a mental coordinate in each of the mouth's location (hard palate; soft palate; against the left and right cheek) as a result of the tongue's movement in each direction. If the patient is not being successful in moving their tongue in one of these directions, then the feedback will deliver a white-filled tongue which means downregulation or decrease in BOLD signal of the region functionally associated with the lesioned region instead of upregulation (increased activation) of the functionally associated with the lesioned area) when the tongue is filled in red. Changing at least one motor behavior of the tongue may refer to improved function in tongue movement relative to the patient's condition prior to any neurofeedback training.

It will be apparent to a skilled person that the method may be performed in a variety of sessions, each session including the BOLD signal of one or more of the activations of brain areas described herein. In various embodiments, the method includes a first session and a second session. The first session may include: measuring a magnitude and a spatial extent of activation in a plurality of brain regions of the patient during movement of the tongue by fMRI; generating a map of an individualized network of brain regions that control tongue movement; and identifying at least one intact brain region functionally associated with the lesioned brain region based on the map of the individualized network of brain regions. The second session, and in various embodiments subsequent sessions, may include further monitoring the level of activation in the plurality of brain regions of the patient by functional magnetic resonance imaging (fMRI); tracking at least one tongue movement of the patient; providing neuronal feedback to the patient based on the level of activation of the brain region identified and the at least one movement of the tongue, and instructing the patient to change at least one motor behavior of the tongue based on the neuronal feedback, thereby treating the disorder.

### Tangible, non-transitory computer readable medium

In another aspect, the invention provides a tangible, non-transitory computer-readable medium comprising computer-program instructions for implementing a method of treating a swallowing, mastication, or speech articulation disorder associated with a lesioned brain region in a patient, the method comprising: measuring a magnitude and a spatial extent of activation in a plurality of brain regions of the patient during movement of the tongue by fMRI; generating a map of an individualized network of brain regions that control tongue movement; identifying at least one intact brain region functionally associated with the lesioned brain region based on the map of the individualized network of brain regions; tracking at least one tongue movement of the patient; providing neuronal feedback to the patient based on the level of activation of the brain region identified and the at least one movement of the tongue, and instructing the patient to change at least one motor behavior of the tongue based on the blood-oxygen-level-dependent (BOLD) signal which reflects the neuronal feedback, thereby treating the disorder.

### MRI-compatible measurement device for the tongue

In another aspect, provided is an MRI-compatible tongue measurement device having a mouthpiece with multiple degrees of freedom to incorporate any tongue movement: an incisor-adjacent sensor sheet, a hard palate sensor sheet, a soft palate sensor sheet, and at least one sensor mounted on each of the incisor-adjacent sensor sheet, the soft palate sensor sheet and the hard palate sensor sheet. The MRI-compatible tongue measurement device allows measurement of tongue movement and, in various embodiments, the force exerted by the tongue. In various embodiments, the invention provides an MRI-compatible tongue fiber optic sensor (200) having a mouthpiece such as that described above (202) and a fiber-optic cable (204) configured for connecting a camera (208) to the mouthpiece (202). The MRI-compatible tongue sensor needs to be free from magnetic components (*e.g.,* ferrous metals) that may interfere with an MRI scanner. In various embodiments, what will reside in the patient's mouth is a very thin and soft mouthpiece. In various embodiments, the mouthpiece will have holes, each hole may have a diameter equal to or less than 50 microns configured such that a fiber-optic sensor may be inserted and sensors that may record movements of the tongue.

The sensor plates can be constructed from non-magnetic material compatible with the MRI scanner's magnetic field. In various embodiments, the material will be disposable. In various embodiments, the sensor sheet can be made of food-safe silicon and the like.

The sensor sheets can have a size and shape that allows them to fit within the mouth of the patient. The incisor-adjacent sensor sheet can be configured to fit into the region of the patient's mouth between the tongue and the incisors. The hard and soft palate sheets can be connected to the incisor adjacent sensor sheet and configured to rest against the hard and soft palate respectively. In various embodiments, the hard and soft palate sheets are shaped to substantially conform to the palate against which they are designed to rest.

The sensors can be distributed between the sensor sheets. In various embodiments, the MRI-compatible tongue measurement device can include up to 20 sensors total (*e.g.,* five sensors can be mounted on the right side of the adjacent sensor sheet and five sensors can be mounted on the left side of the sensor, five sensors can be mounted on the soft palate sensor sheet, and five sensors can be mounted on the hard palate sensor sheet; or two sensors can be mounted in the middle of the mouthpiece and one sensor on the right side and one sensor of the left side).

Fiber-optic sensors mounted on these sheets/plates may measure pressure/force of the tongue movement and, in turn, provide a reconstruction of the tongue movement, since pressure can be conceptualized as the movement divided by the velocity of the tongue and ultimately tongue strength. In various embodiments, the sensor or sensors are pressure sensors.

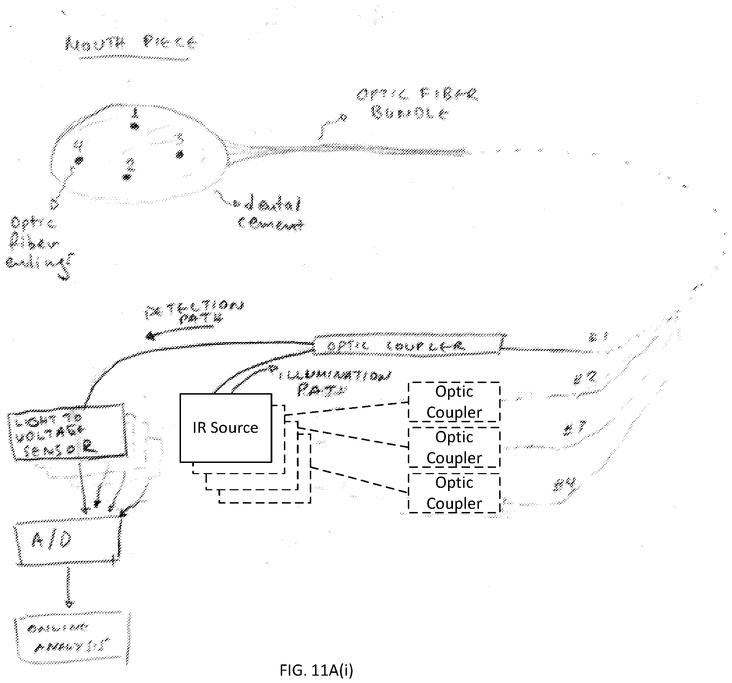

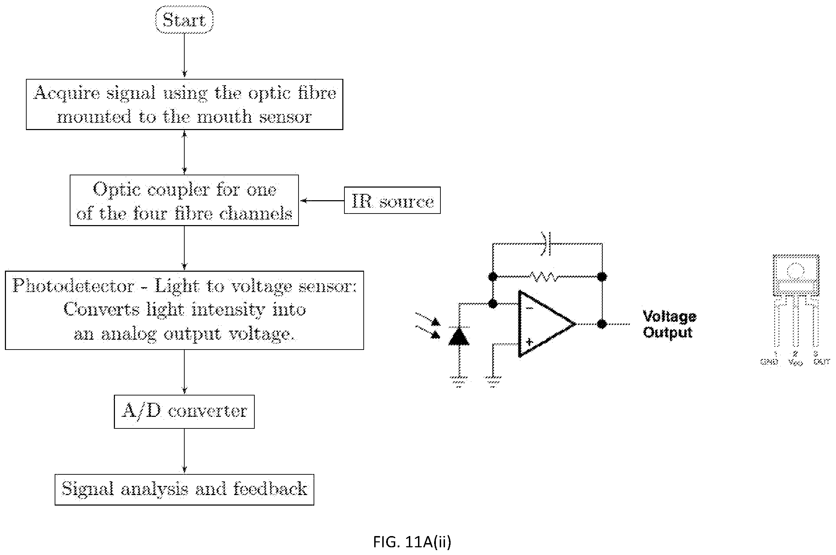

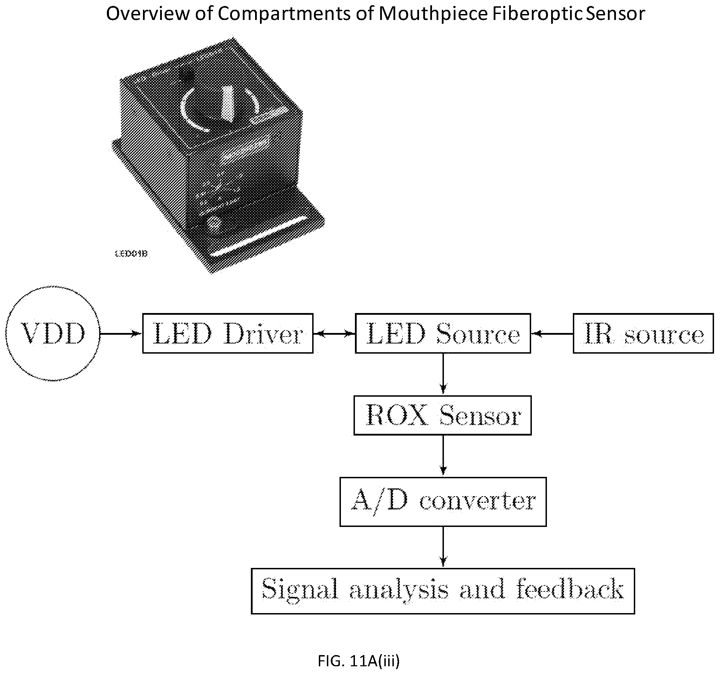

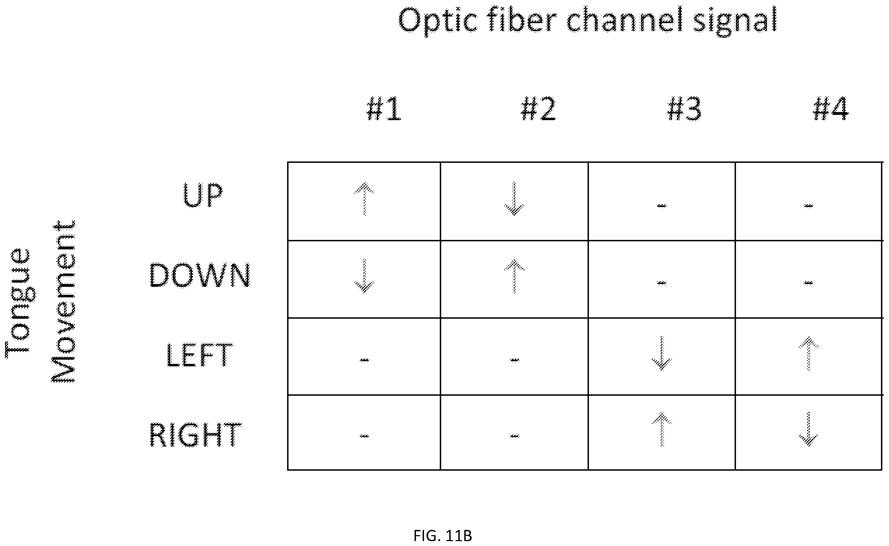

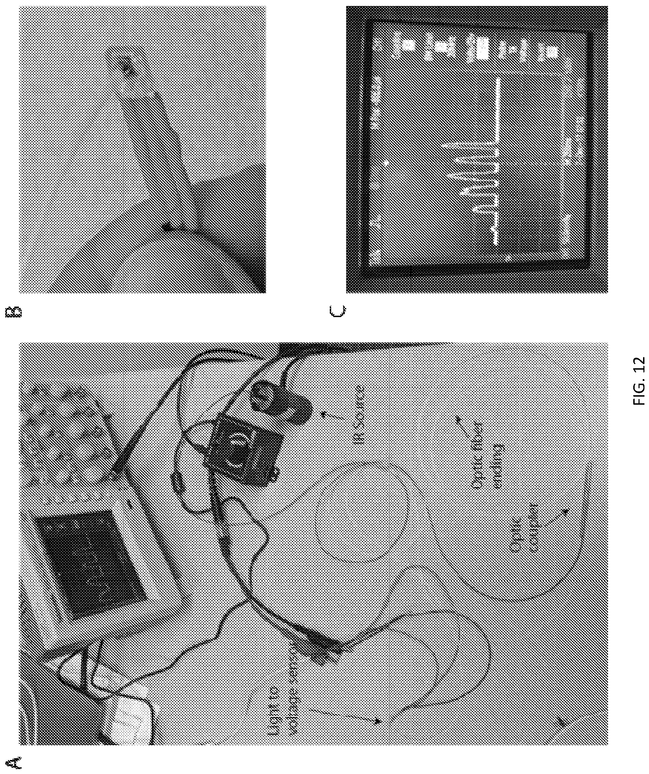

In various embodiments, as illustrated in FIGS. 11A(i)-11A(iii), the sensors may be the ends of fiber optic cables that are embedded in the mouthpiece. Each fiber optic cable may be connected to an optic coupler and to an IR source as well as a light to voltage sensor (FIG. 12). Sensors may send signals through different channels to facilitate tracking of tongue movement. In various embodiments, the sensors use a photodetector to detect reflected light emitted from the optic fiber that will vary based on the distance from an object (*i.e.,* the tongue) to the optical fiber ending. The reflected light can be detected by a photodetector. Different tongue movements will lead to different signals in the channels. Signals from a four channel embodiment are illustrated in FIG. 11B.

In various embodiments, the fiber-optic sensor will capture translation and rotation of tongue movement such as:
- left-right tongue movement (vertical tongue movement), which can involve translation in the X-axis or rotation in the Z-axis
- up-down tongue movement (horizontal tongue movement), which can involve translation in the Z-axis or rotation in the X-axis
- back-forth tongue movement (horizontal tongue movement), which can involve translation in the Y-axis or rotation in the Y-axis.

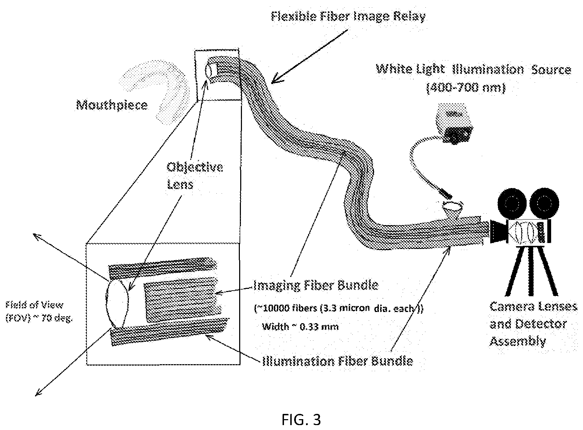

In various embodiments, referring now to FIG. 3, the MRI-compatible measurement device further comprises a camera as part of the sensor connected to the fiber-optic cable (204) and includes an illumination fiber bundle (210) configured for connection to the illumination source (206); and an imaging fiber bundle (212) configured for connection to the external components of the sensor (208) and to a computer, a display or another device that facilitates interpretation of the signal. In various embodiments, the illumination fiber bundle (210) directs light from the illumination source to the interior of the patient's mouth. The imaging fiber bundle (212) allows the sensor to capture images of the interior of the patient's mouth, in particular the movement of the tongue. In various embodiments, the fiber-optic cable (204) is long enough to reach the sensor (208) when the sensor (208) is placed at a safe distance from the MRI scanner (*e.g.,* five meters, ten meters or more).

The illumination source (206) provides light to facilitate monitoring the movement of the tongue. In various embodiments, the illumination source (206) emits light with a wavelength of 400-700 nm.

A skilled person will immediately understand that a variety of configurations for the mouthpiece (202) and the fiber-optic cable (204) are possible. For example, the illumination fiber bundle (210) and the imaging fiber bundle (212) may be separate cables or may be portions of the same cable. In various embodiments, the fiber-optic cable (204) is connected to the mouthpiece (202) at an incisor adjacent region, meaning that the fiber-optic cable (204) is connected to the portion of the mouthpiece designed to rest in proximity to the patient's incisors when placed in the patient's mouth. Mouthpiece (202) can be any device capable of being held in a fixed location in a subject's mouth. In some embodiments, the mouthpiece (202) approximates the subject's denture. In various embodiments, the measurement device may include the sensors and sensor sheets described above as well as the camera and illumination source or any combination of the features described herein.

### System including an MRI-compatible tongue fiber optic sensor and a controller

In a further aspect, the invention provides a system, including an MRI-compatible tongue fiber optic sensor or an MRI compatible tongue sensor (200), as described above, and a controller (300) programmed to: measure a magnitude and a spatial extent of activation in a plurality of brain regions of the patient during movement of the tongue, by fMRI; generate a map of an individualized network of brain regions that control tongue movement; identify at least one intact brain region functionally associated with the lesioned brain region based on the map of the individualized network of brain regions; track at least one tongue movement of the patient; provide neuronal feedback to the patient based on the level of activation of the brain region or network of regions identified and the at least one movement of the tongue, and instruct the patient to change at least one motor behavior of the tongue based on the neuronal feedback, thereby treating the disorder; and a display (400) configured to provide feedback to the patient. The system can include an MRI scanner (500).

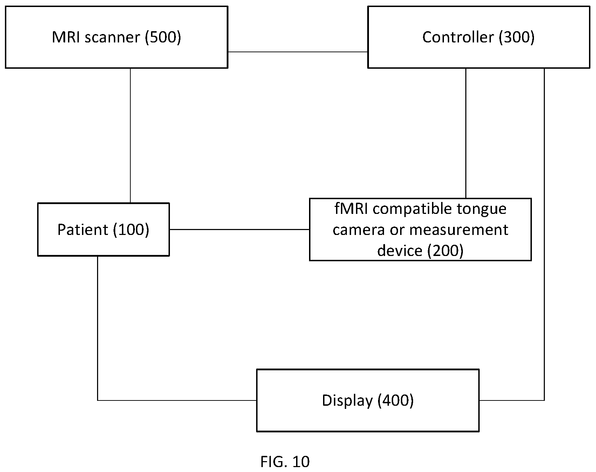

FIG. 10 shows the MRI-compatible tongue sensor measurement device (200) can be placed within the mouth of the patient during operation of the system. Data about the movement of the tongue is sent to the controller (300) for processing. The controller (300) can be a computer loaded with software designed to facilitate the performance of the method. The controller (300) can be connected to the MRI scanner (500). The display (400) can be placed within the view and/or hearing of the patient (100), as appropriate for the provision of neuronal feedback. The display (400) can be and/or include one or more of a computer monitor or screen, or can be a projector, MRI compatible mirror, mirror on which a computer screen is presented via a projector. The connections between the controller (300), MRI compatible tongue measurement device or tongue sensor (200) and the MRI scanner (500) can be wired or standalone wireless connections.

### EXAMPLES

The invention is now described with reference to the following Examples. These Examples are provided for the purpose of illustration only, and the invention is not limited to these examples, but rather encompasses all variations that are evident as a result of the teachings provided herein.

Patients with: (i) hypoglossal, glossopharyngeal nerve (supra- or infra-nuclear) injury; (ii) hemiglossectomy with hypoglossal anastomosis; (iii) isolated hypoglossal nerve palsy (HNP), or neurapraxia (a rare postoperative complication after airway management, which results in causes ipsilateral tongue deviation), (iv) injury to the marginal mandibular branch of the facial nerve; and, (v) late-radiation-associated dysphagia with low cranial nerve neuropathy are enrolled. Hypoglossal nerve injury is multifactorial and can occur at the level of each of the following segments of this nerve: (i) medullary segment, as a result of glioma, infarction, hemorrhage; (ii) cisternal segment, as a result of aneurysm, basilar ectasia, meningioma, rheumatoid arthritis; (iii) skull base segment, as a result of nasopharyngeal carcinoma and other types of tumors; (iv) carotid space segment, as a result of nodal carcinoma, and carotid dissection; and (v) sublingual segment, as a result of carcinoma infection. The primary outcome is to enhance controlled and voluntary movement of the tongue and, in turn, swallowing, mastication, and speech articulation in patients with supranuclear or infranuclear lower cranial nerve lesions, specifically, Hp, Gp, or facial by applying a novel, non-invasive real-time fMRI neurofeedback approach aiming to strengthen Hg-, Gp-bypassing pathways that originate at the level of the medulla oblongata (nucleus ambiguus, nucleus solitarius, spinal nucleus of the trigeminal).

The secondary outcome is to improve speech articulation as a result of tongue movement neuro-rehabilitation (FIG. 1). The method applies individualized real-time fMRI neurofeedback for the neuro-rehabilitation of neurological injury (motor and visual), not only at the central but also peripheral (distal) nervous system injury (supra- or infra-nuclear nerve injury) and delivers longitudinal training until performance is enhanced and maintained.

Rt-fMRI nFb training enhances tongue movement and speech articulation in patients with Hg and Gp lesions. Improved voluntary and controlled tongue movement, swallowing, mastication and, speech articulation performance will be mediated via strengthening of functional pathways that bypass the area of Hg and Gp injury to reorganize pathways in the somototopic areas of the motor and somatosensory networks. The neuro-rehabilitative success of rt-fMRI nFb is based on this continuous pairing between fine motor control of tongue movement and mental imagery of the tongue's movement, which stimulates associative learning and enhances long-term potentiation, a measure of long-term synaptic plasticity, and a predictor of the haemodynamic response.

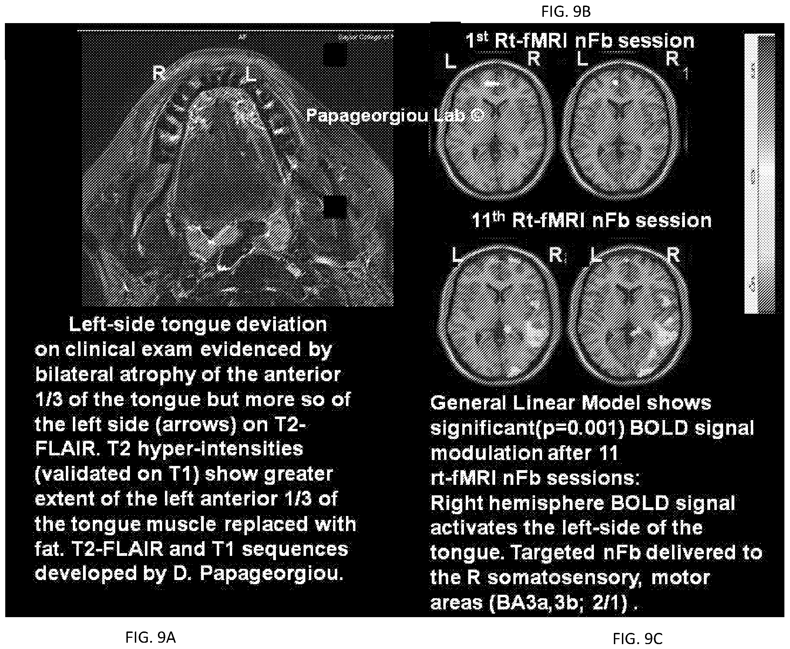

The continuous tongue motor training to improve control of the tongue movement ("bottom-up"), while cortical and subcortical areas (cerebellum/dentate; insula; basal ganglia; medulla; somatosensory areas; thalamus) were generated through support vector machine analysis (FIG. 7) are maximally upregulated via the repetitive pairing of mental imagery of the tongue movement ("top-down") will induce associative learning and promote plasticity in surviving, hypoglossal- and glossopharyngeal-bypassing pathways that originate from the medulla. This training has been applied during real-time fMRI neurofeedback (FIGS. 7 and 9A-9C). Thus, the immediate goal is to capitalize on intact functional branches of these cranial nerves, with outputs at the precentral gyrus' level (BA4) via upper motor neurons, where the corticobulbar input to the Hg nucleus arises and strengthens them via neurofeedback. rt-fMRI nFb is used to train patients to upregulate cortical and subcortical regions of interest, innervated by branches of the Hg/CNXII, and Gp/CNIX nerves.

The data shows that targeting S1, specifically Brodmann Area (BA) 3a (nociceptive ROI), BA 3B (nociceptive and somatosensory ROI), BA 2/1 (nociceptive and somatosensory ROI), and BA 4 (somatomotor), as well as the cerebellum/dentate (planning, initiation and control of voluntary movements), insula, basal ganglia (somatomotor), thalamus via rt-fMRI nFb, for the purpose of delivering efferent bypassing input pathways to the anterior part of the tongue, can enhance tongue movement performance and, in turn, swallowing and speech intelligibility. These data show success in being able to neuromodulate the BOLD signal by targeting BA area 3a, 3b, and BA 2/1 of the motor network of an adult patient with impaired tongue movement (FIG. 9A), along with dysarthria, and swallowing, as a result of a neonatal injury. Upon completion of 11 controlled tongue movement rt-fMRI nFb sessions, increased right hemisphere BOLD modulation was induced (FIGS. 9B and 9C; the right hypoglossal nerve crosses to the left side of the tongue, which decodes for Right/Left tongue movement by left (red)/right (cyan) hemisphere) accompanied by enhanced speech intelligibility. The longitudinal motor rt-fMRI nFb neuromodulation data is significant, especially because the injury is of neonatal origin. The next target was the medulla oblongata, where sensory and motor neurons (nerve cells) from the forebrain and midbrain travel through the medulla, as well as the nuclei of Hp and Gp based on a network of regions mapped using classification machine algorithms (FIGS. 6 and 7).

fMRI data collected from a patient with anterior bilateral atrophy of the tongue as a result of a lesion to the hypoglossal nerve shows a unique and individualized cortical and subcortical network of regions of activation (FIGS. 7 and 8). The unique network of regions during left- and right-, up-, and down-ward voluntary and controlled tongue movement, generated via support vector machine (FIG. 7) and general linear model (FIG. 8) computations are the following: cerebellum/dentate; insula; basal ganglia (caudate; putamen; globus pallidus; lentiform nucleus); medulla; somatosensory areas (BA3a/3b/1/2); thalamus. The patient's preliminary data has also generated occipital lobe activation, specifically primary visual cortex which suggests mental imagery of the tongue movement (FIG. 8). The patient has reported that the ability to move the tongue in a controlled and timely manner (up; down; left; right tongue movement) inside the fMRI environment is facilitated by employing mental imagery: visualization of the tongue movement touching the hard palate to achieve upwards motion, touching the soft palate to achieve downwards motion and left and right movement of the tongue voluntary and controlled movement of the tongue localization. Data of healthy subjects during performance of tongue movement inside the fMRI environment has not generated primary visual cortex activation.

The goal is to understand the learning mechanisms that occur using machine learning approaches. Specifically, (i) to develop a computational model using the longitudinal data generated as a function of learning that will allow characterization of the type of associative learning; (ii) to examine whether there is dependency of the learning mechanisms that occur in response to the type of brain function in order to neuromodulate and eventually neurorehabilitate; (iii) to examine the learning mechanisms induced by this training approach in healthy subjects (the goal of which is to increase performance) as opposed to the learning mechanisms that take place in patients (the goal of which is to induce recovery of the lesioned brain function by bypassing injured pathways and capitalizing on intact but functionally associated to those that have sustained injury). Understanding of the learning mechanisms induced in visual, motor or pain networks will be enhanced by combining electroencephalography (EEG) during real-time fMRI neurofeedback. EEG measurements will enhance the temporal resolution and thus, are complimentary to fMRI measurements, which offer excellent spatial resolution.

The goal is to use rt-fMRI nFb to elucidate the mechanism(s) of increasing somatosensation and somatomotor activity in patients with Hp- and Gp-injury as a result of CNS- (supranuclear) or PNS- (infranuclear) nerve injury. The approach developed to neuromodulate sensory and motor control of the tongue is pioneering and cutting-edge because it is: (i) non-invasive; (ii) plasticity promoting, and (iii) individualized, as it can target specific spatial extents of each patient's ROIs at the CNS level (e.g., supranuclear lower cranial nerve lesions) and PNS level (infranuclear - below the brainstem lower cranial nerve lesions).

Cortical and subcortical plasticity can be induced along specific neural pathways. This can be achieved by voluntarily modulating the activity of selected brain areas that serve as inputs or outputs of these pathways, via neuro-rehabilitative training. The theoretical framework of learning mechanisms induced by neurofeedback of visual motion perception is based on associative learning, specifically in both the classical and operant conditioning forms. Specifically, a BA3b neuron receives input during the presentation of an unconditioned stimulus (US), such as RDKs moving in a coherent direction. This will generate hV5/MT+ upregulation in response to direction selectivity of coherent motion. Classical conditioning mechanisms can transfer the MT upregulation, which will lead to increased visual motion performance during the presence of a conditioned CS, such as the neurofeedback interface characterized by a white tongue, which denotes decreased BOLD signal of BA3b and in turn, decreased movement of the tongue (decreased dynamic range of the tongue movement). But how is this conditioned response strengthened? The operant learning theory can be used to explain the strengthening of the conditioned response; i.e., the reinforcement stimulus that is presented in the form of neurofeedback will control the probability of the occurrence and magnitude of a conditioned physiological response. In other words, the consequence of a response's strength (increase or decrease) is a function of the reinforcement stimulus (positive or negative) or, punishment (positive or negative) that it can elicit. There is also the possibility that neurofeedback may be underlined by the engagement of representational learning mechanisms. This type of learning refers to Bayesian formed expectancies (priors), which are computationally formed by experience and can be computationally retrieved in a top-down manner by higher-order cognitive functions, such as memory and attention networks.

The goal is to understand how the behavioral framework of associative learning translate to the neuronal mechanisms of plasticity during neurofeedback. As of now, no attempt has been made yet to answer this question. Therefore, the aims are: (i) to develop a computational model using the longitudinal data generated as a function of learning that will allow characterization of the type of associative learning; (ii) to examine whether the learning mechanisms that occur are dependant in response to the type of brain function intended to neuromodulate and eventually neurorehabilitate; (iii) to examine the learning mechanisms induced by this training approach in healthy subjects as opposed to the induction of learning that evolves in patients. While the goal for the induction of learning in healthy subjects and patient populations is to increase performance, the approach and the methodology to accomplish this, induces learning via engaging different neural pathways and thus, mechanisms: (i) in healthy subjects via the strengthening of already intact pathways, accomplished via upregulation of the BOLD signal (relative to baseline) to successfully perform a task of a given difficulty; (ii) in patient populations via bypassing of injured pathways and capitalizing on intact but functionally associated to those that have sustained injury to induce recovery of the lesioned brain function. The magnitude of the BOLD signal in patients will be dependent on the type of alternate connections to the functionally redundant pathways that will be established. Understanding of the learning mechanisms induced in somatomotor and somatosensory networks will be enhanced by combining electroencephalography (EEG) during real-time fMRI neurofeedback. EEG measurements will enhance the temporal resolution of the data, since fMRI measurements offer excellent spatial resolution but suffer from poor temporal resolution.

One of the goals of developing a computational model is to predict the learning mechanism of the BOLD neuromodulation generated as the result of the longitudinal rt-fMRI nFb data:
(i) A Hebbian learning mechanism will refer to a continuous interaction between cortical ROIs that correspond to specific inputs (excitatory presynaptic level) and outputs (excitatory postsynaptic level) of neurons, associated with somatomotor and somatosensory activation, and the strengthening and increased efficacy of these ROIs' outputs is accomplished via highly correlated activations. For example, subject 3 (FIG. 6), demonstrates activation of somatosensory and somatomotor, and proprioceptive awareness/attentional ROIs in response to tongue movement, such as activation of the insula and dentate, which are highly correlated. Behavioral performance such as measurement of tongue movement is positively correlated with the ROIs' BOLD signal. The goal is to decipher whether this correlation remains consistent as a function of learning induced by the purposeful induction of real-time fMRI neurofeedback.
(ii) A anti-Hebbian learning mechanism will refer to a continuous interaction between cortical ROIs that correspond to specific inputs (inhibitory presynaptic level) and outputs (postsynaptic level) of neurons, associated with somatomotor and somatosensory activation and the strengthening of these ROIs is accomplished via highly anti-correlated activations. However, increased connection strength between the ROIs corresponds to decreased efficiency in eliciting a response. For example, patient data (FIG. 7) demonstrates activation of the occipital lobe as a mental imagery strategy in response to achieving movement of the tongue on the left-right plane. The hypothesis is that as a function of training, the activation of the cortical and subcortical somatosensory and somatomotor networks will strengthen in response to tongue movement, as a result of accomplishing voluntary and controlled movement of the tongue more efficiently (FIG. 7).
(iii) A combination of a Hebbian and an anti-Hebbian learning mechanism (interaction) where the sum of inputs are achieved by feedforward synaptic weights (Hebbian) while outputs are weighted by lateral connections (anti-Hebbian). A combination of these two mechanisms can be the described by upregulation of the targeted ROI and generation of increased activity in the pathway the ROI belongs to, but by downregulation in an indirect or compensatory pathway that controls movement of the tongue. An example of a combination of the two would be the decreased activity of BA3 compared to the greater activation of frontal motor processing areas, such as inferior frontal gyrus, or, middle frontal gyrus, or insula. Such activation pattern may be typical during neurofeedback training to achieve enhanced performance of tongue movement in a voluntary and controlled fashion (FIG. 9C).

Information about the changes in the magnitude of the BOLD signal is combined computationally - the prior, each weight update depends on the up- or down-regulation of the ROI that targeted for neuromodulation - as a function of learning based on machine learning and Bayesian computations. It is advantageous to increasingly rely on prior knowledge in the real-time fMRI setting because when new observations become available, the previous posterior BOLD distribution can be continuously updated. Estimation of the prior, meaning the probability that the ROI reaches maximum upregulation in more than 50% of the trials or 25% trials will be determined: i.e., what would be the magnitude of a prior that would be considered successful BOLD signal modulation for neurofeedback delivery in the targeted ROI: (i) across different brain functions (somatosensory, somatomotor, proprioceptive awareness, attention-associated and other ROIs as shown in FIGS. 1, 2 and 6); (ii) patients vs healthy subjects; (iii) long-term (for e.g., neonatal) vs acute injury (for e.g., a few years after one sustains a stroke or traumatic brain injury) for example, the probability that the ROI reaches increased activity more than 50% of the trials or 25% trials, etc.).

Identifying the mechanisms that guide neuromodulation as a function of longitudinal learning using real-time fMRI neurofeedback will help develop hypotheses for future sensory and motor neuro-rehabilitation, and, in turn, pain neuro-rehabilitation because the somatosensory and somatomotor areas are also part of the pain brain network (somatosensory, basal ganglia, thalamus, insula, cerebellum/dentate, pons). In other words, the ROIs and pathways responsible for BOLD modulation, such as proprioceptive awareness, attention-associated, somatosensory, somatomotor will be elucidated with the goal to induce changes in redundant somatosensory and somatomotor areas (perception and sensation areas) for the purpose of neuro-rehabilitation.

Structural and functional validation of the real-time fMRI neurofeedback approach: Pre- and post-real time fMRI neurofeedback sessions, the induced changes in the magnitude and spatial extent of the BOLD signal will be validated with structural and functional methods in pathways of ROIs that are included in the ascending and descending spinothalamic pathways, as well as with ROIs in spinohypothalamic, and spinoreticular (reticular formation to thalamus) corticonuclear (corticobulbar), trigeminal and solitary pathways, as different types of cranial lesions can modulate different pain, sensory, and motor pathways.

Computational optimization for magnitude and spatial extent of neurofeedback delivery: ROIs - unique to each patient - with the maximum or minimum activity depending on the sensory/motor/pain pathway. Functional scans are selected for neuromodulation. This optimization step is important so that neurofeedback delivery is based on the maximum/minimum spatial extent of the BOLD signal unique to each patient's circuitry. For example, a patient's pain circuitry may include increased activation of ascending pain pathways, such as insula and anterior cingulate, while another patient's pain matrix may include increased activation of ROIs that belong to the ascending pain pathway, but also moderate activation of descending pain pathway ROIs, such as periaqueductal cortex activity. These computations are based on machine learning and support vector machine (SVM) methods. The goal is to target the neurofeedback to those ROIs unique to a patient's functional circuitry as defined by what is intact after taking into consideration the location of the lesion and its spatial extent, so that the neurofeedback is as individualized as possible based on the spatial extents of each pain patient's functional neuroimaging.

Computational optimization for temporal neurofeedback delivery: The temporal neurofeedback delivery is optimized computationally by: (i) improving the signal-to-noise ratio; (ii) accounting for the delay of the hemodynamic response; (iii) minimizing cognitive recruitment by allowing the patient enough time to modulate the ROI's BOLD signal intensity; (iv) accounting for other confounds, such as the subject engaging in irrelevant strategies by performing the task and completing the trials, independent from the evaluation of the neurofeedback.

Combination of a high temporal resolution modality with a high spatial resolution modality to elucidate plasticity mechanisms: Adequate spatial resolution using rt-fMRI nFb is applied for the purposeful induction of neuromodulation to somatomotor and somatosensory areas. These areas are innervated by cranial nerves (Hp and Gp), which originate at the brainstem level with outputs to somatosensory regions (postcentral gyrus), such as BA3b. This is combined with high temporal resolution modalities, such as electroencephalography (EEG) and electromyography (EMG) with the goal to elucidate the mechanisms that guide plasticity. EEG reflects voltages generated by inhibitory but mostly excitatory postsynaptic potentials from apical dendrites of neocortical pyramidal cells. Long-term potentiation (LTP) is a measure of synaptic plasticity which underlines learning and memory formation, extensively studied at the molecular level in animals. However, for the first time, LTP is studied using EEG while inducing plasticity using a longitudinal real-time fMRI neurofeedback experimental design in a neurologic patient population.

Motor unit action potentials (MUAP) are measured during swallowing using EMG, while inducing neuro-modulation via real-time fMRI neurofeedback. These additional measures will allow elucidation of the mechanisms of learning that result in the neuromodulation of ROIs along the ascending and/or descending somatosensory, somatomotor, and nociceptive pathways. EMG is also applied.

Computational approach for neurofeedback signal: Neurofeedback delivery is based on: (i) the anatomical co-ordinates of somatosensory and somatomotor ROIs as reported in the literature; (ii) other individualized ROIs generated during controlled tongue movement when no neurofeedback is delivered (functional localization during fMRI scans) using machine learning (support vector machine) analyses (FIGS. 6 and 7); and (ii) the spatial extents generated by the acquisition of a functional localizer task that measures the BOLD activation during controlled tongue movement without neurofeedback delivery. This is a powerful strategy because neurofeedback is based not only on validated anatomical, atlas-based landmarks, but also on the individualized magnitude and spatial extent of somatosensory, somatomotor, and pain-associated ROIs, unique to each patient and the specific location and extent of their pathology (e.g., supranuclear versus infranuclear cranial nerve lesion). To generate and deliver neurofeedback signal from each ROI, an incremental and updated mean or, median based on the general linear model computed in each voxel that belongs to each ROI is applied.

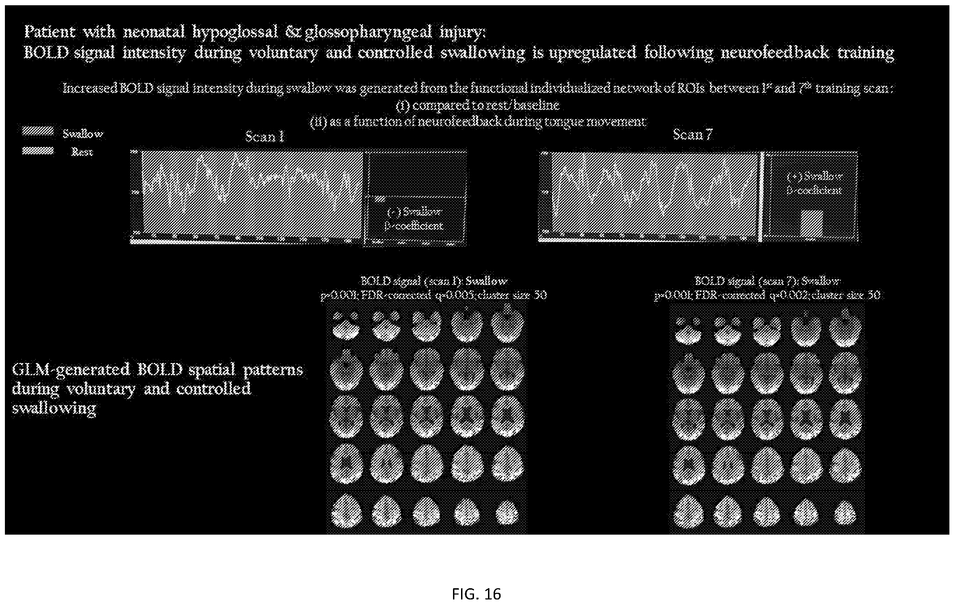

The data presented here demonstrates success in being able to neuromodulate the BOLD signal of the motor network of an adult patient with impaired tongue movement (FIG. 9A), along with dysarthria, and swallowing (FIG. 16), as a result of a neonatal injury. Upon completion of 7 for swallowing or 12 for controlled tongue movement rt-fMRI nFb sessions (because the injury is congenital and thus, the training is much lengthier than a patient with an acquired injury), increased right hemisphere BOLD modulation was induced (FIGS. 9B and 9C; the right hypoglossal nerve crosses to the left side of the tongue), accompanied by enhanced speech intelligibility. The longitudinal motor rt-fMRI nFb neuromodulation data is significant, especially because the injury is of neonatal origin. The persistence of pain neuromodulation effects are examined by measuring the magnitude and spatial extent of the BOLD, DTI and morphometry parameters at 6 months, 1 year, 1.5 years, 2 years, 3 years, 4 years after acquisition of the last neurofeedback scan when BOLD plateau has been reached.

Development of a fiber-optic sensor or accelerometer inside the MR-environment for measuring tongue movement (velocity) in patients with central (upper motor neuron disease; supranuclear lesion) or peripheral nervous system (lower motor neuron disease; infranuclear lesion) disease.

An intra-oral fiber-optic sensor is developed to record tongue movement while the patient is inside the MR environment performing the tongue fMRI and real-time fMRI neurofeedback task. This device is used in patients with central (upper motor neuron disease characterized by supranuclear lesions to the hypoglossal and/or glossopharyngeal nerves) and peripheral nervous system (lower motor neuron disease characterized by infranuclear lesions to the hypoglossal and/or glossopharyngeal nerves) injuries to record tongue movement and provide motor control feedback via machine learning algorithms. The magnitude of tongue movement is: (i) decreased in patients (head and neck cancer patients after radiotherapy-based treatment; lower cranial nerve palsies, such as supranuclear or infranuclear glossopharyngeal and/or hypoglossal lesions) in comparison to healthy subjects; (ii) decreased as a function of increased tongue deviation to the vertical and/or horizontal planes.

The intra-oral tongue sensor or accelerometer device includes the following features:
(1) The MR-compatible fiber-optic sensor or accelerometer is sterilizable.
(2) Sensors will be placed on a MR-compatible mouthpiece and on a sensor sheet along the palatal plate and surface, which will quantify the tongue velocity (pattern of tongue movement). The placement of a total of 3-4 sensors is important as the patients' tongue deviates either to the left or to the right (it is not isometric) and can thus, mask the direction of tongue movement in the vertical and/or horizontal planes (see item 3).
(3) The fiber-optic sensor or accelerometer captures Translation and Rotation of tongue movement:
   - left-right tongue movement (vertical tongue movement), which can involve Translation in the X-axis or Rotation in the Z-axis
   - up-down tongue movement (horizontal tongue movement), which can involve Translation in the Z-axis or Rotation in the X-axis
   - back-forth tongue movement (horizontal tongue movement), which can involve Translation in the Y- axis or Rotation in the Y-axis
(4) The software that records the movement provides a reconstruction of the tongue movement in all planes. Placing 3-4 microscopic sensors distributed to the parts of the mouthpiece that is placed adjacent to the hard palate, to the soft palate, and to the right and left incisors will facilitate recording tongue deviation and thus, enable reconstruction of tongue movement. The goal is that the accelerometer makes highly sensitive measurements with regards to recording any pressure applied directly from the tongue to the sensor(s), as pressure can be conceptualized as the movement/velocity and ultimately tongue strength.

The long-term objectives are (i) to provide motor control feedback via machine learning algorithms, such as support vector machine, bayesian, and neural network computations; and (ii) to correlate neuromodulation of the blood-oxygen-level-dependent (BOLD) fMRI signal with tongue movement as a function of neuro-rehabilitation of motor control during the application of real-time functional MRI neurofeedback sessions longitudinally and computer vision feedback, respectively. Specifically, motor feedback of the magnitude of the tongue movement is provided via machine learning algorithms, such as support vector machine and Bayesian computations. BOLD neuromodulation in the individualized network of areas generated by tongue movement is correlated with changes in actual tongue motor control recorded by the tongue force generation device.

Acquire behavioral (outside the MRI scanner) measurements of tongue strength before and after real-time fMRI: Tongue strength is measured since tongue weakness is a sequelae of hypoglossal and glossopharyngeal lesions. These measurements are acquired using the IOPI system (Iowa Oral Performance Instrument). The IOPI device measures tongue strength as a function of maximum pressure by placing a squeezing bulb against the hard palate: i.e., "tongue elevation strength is the maximum pressure of the tongue pressing against the hard palate", http://www.iopimedical.com/Tongue_Strength.html)

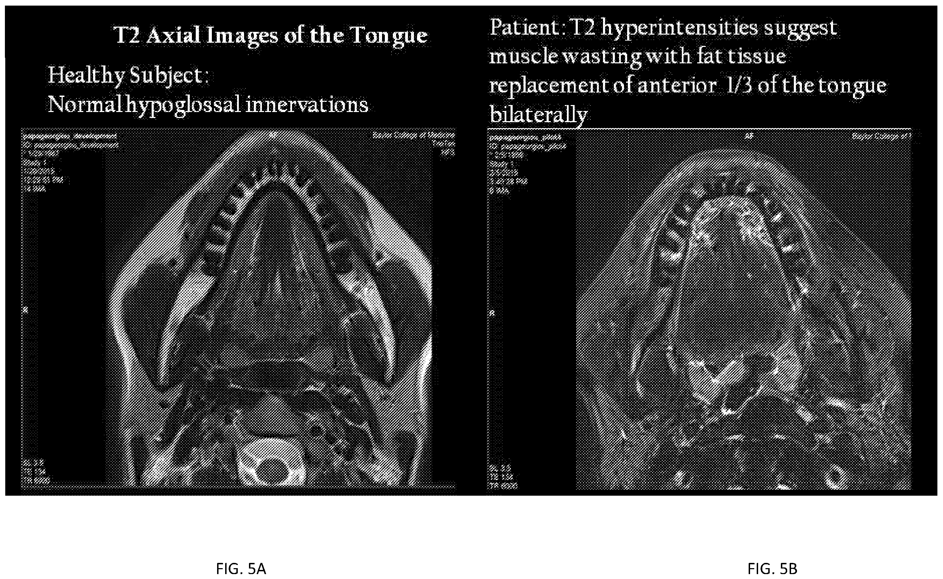

Acquire innovative fMRI and real-time fMRI neurofeedback sequences of the tongue: As an adjunct and/or alternative to the fiberoptic sensor and/or accelerometer of the tongue, fMRI sequences of the tongue are acquired while the subject is performing the same task as the one during the acquisition of the brain fMRI. This is feasible because MRI sequences of the tongue have already been developed and acquired (FIG. 5). Tongue real-time fMRI neurofeedback is performed to modulate tongue motor control by employing the same task as the one the subject/patient performs during real-time fMRI neurofeedback to the cortical areas that are associated with tongue movement.

## Claims

1. A system comprising:
an MRI-compatible tongue measurement device, comprising:
a mouthpiece, and
a fiber optic cable configured for connecting an illumination source and a sensor to the mouthpiece;
a controller programmed to:
measure a magnitude and a spatial extent of activation in a plurality of brain regions of the patient during movement of the tongue, by fMRI;
generate a map of an individualized network of brain regions that control tongue movement based on the measurements of the patient's brain during movement of the tongue;
identify at least one intact brain region functionally associated with a lesioned brain region based on the map of the individualized network of brain regions, wherein the intact region is involved in the movement of the tongue;
track at least one tongue movement of the patient;
provide neurofeedback to the patient based on the level of activation of the brain region identified and the at least one movement of the tongue; and
instruct the patient to change at least one motor behavior of the tongue based on the neurofeedback, thereby treating the disorder; and
a display configured to provide feedback to the patient.

2. The system according to claim 1, further comprising:
an MRI scanner.

3. The system according to claim 1, wherein the at least one intact brain region is selected from the group consisting of Brodmann Area 3A, Brodmann Area 3B, Brodmann Area 2/1, Brodmann Area 4, Brodmann Area 6, pre-central gyrus, the cerebellum, dentate nucleus, culmen, insula, basal ganglia, Brodmann Area 9, inferior frontal gyrus, middle frontal gyrus, medial frontal gyrus and thalamus.

4. The system according to claim 1, wherein the MRI-compatible tongue measurement device is an
MRI-compatible tongue sensor, wherein the at least one tongue movement is tracked using the MRI-compatible tongue sensor.

5. The system according to claim 1, wherein the controller is further programmed to implement a first session and at least a second session:
wherein the first session comprises:
measuring a magnitude and a spatial extent of activation of the patient's entire brain during movement of the tongue by fMRI;
generating a map of an individualized network of brain regions that control tongue movement; and
identifying at least one intact brain region functionally associated with a lesioned brain region based on the map of the individualized network of brain regions; and
wherein the second session comprises:
further monitoring the level of activation in the plurality of brain regions of the patient by real-time functional magnetic resonance imaging (fMRI);
tracking at least one tongue movement of the patient;
providing neurofeedback to the patient based on the level of activation of the brain region identified and the at least one movement of the tongue; and
instructing the patient to change at least one motor behavior of the tongue based on the neurofeedback, thereby treating the disorder.

6. The system according to claim 1, wherein the patient has a nerve injury.

7. The system according to claim 6, wherein the swallowing, mastication, or speech articulation disorder is caused by glossopharyngeal or hypoglossal nerve damage.

8. The system according to claim 1, wherein the swallowing, mastication, or speech articulation disorder originates at the neuronal level.

9. The system according to claim 1, wherein the nerve damage is at the supranuclear level.

10. The system according to claim 1, wherein the nerve damage is at the infranuclear level of the glossopharyngeal or hypoglossal nerves.

11. The system according to claim 1, wherein the neurofeedback is delivered from the intact region of the brain that controls tongue movement.

12. The system according to claim 1, wherein the fiber optic cable (204) comprises:
an illumination fiber bundle (210) configured for connection to the illumination source (206); and
an imaging fiber bundle (212) configured for connection to the sensor (208).

13. The system according to claim 12, further comprising an illumination source (206) that emits light with a wavelength of 400-700 nm.

14. The system of claim 1, wherein the fiber optic cable is a optic fiber bundle comprising four fiber optic cables (204), each configured for connecting an illumination source (206) and the sensor (208) to the mouthpiece (202), wherein an end of each fiber optic cable (204) is the sensor (208), said sensor (208) is embedded in the mouthpiece (202), and wherein the fiber optic sensor (200) comprises four channels, one for each of the optic fiber cables (204).

15. A tangible, non-transitory computer-readable medium comprising computer-program instructions for implementing, when executed on a computer for controlling the system of claim 1, a method of treating a swallowing, mastication, or speech articulation disorder associated with a lesioned brain region in a patient, the method comprising:
measuring a magnitude and a spatial extent of activation in a plurality of brain regions of the patient during movement of the tongue, by functional magnetic resonance imaging (fMRI);
generating a map of an individualized network of brain regions that control tongue movement based on the measurements of the patient's brain during movement of the tongue;
identifying at least one intact brain region functionally associated with the lesioned brain region based on the map of the individualized network of brain regions, wherein the intact region is involved in the movement of the tongue;
tracking at least one tongue movement of the patient;
providing neurofeedback to the patient based on the level of activation of the brain region identified and the at least one movement of the tongue; and
instructing the patient to change at least one motor behavior of the tongue based on the blood-oxygen-level-dependent (BOLD) signal which reflects the neurofeedback, thereby treating the disorder.

## Patentansprüche

1. System, umfassend:
ein MRT-kompatibles Zungenmessgerät, umfassend:
ein Mundstück und
ein Lichtwellenleiterkabel, das zum Verbinden einer Beleuchtungsquelle und eines Sensors mit dem Mundstück konfiguriert ist,
eine Steuerung, die programmiert ist, um:
eine Größe und eine räumliche Ausdehnung der Aktivierung in einer Vielzahl von Gehirnregionen des Patienten während der Bewegung der Zunge durch fMRT zu messen,
eine Karte eines individualisierten Netzwerks von Gehirnregionen, welche die Bewegung der Zunge steuern, basierend auf den Messungen des Gehirns des Patienten während der Bewegung der Zunge zu erstellen,
mindestens eine intakte Gehirnregion, die funktionell mit einer geschädigten Gehirnregion verbunden ist, basierend auf der Karte des individualisierten Netzwerks von Gehirnregionen zu identifizieren, wobei die intakte Region an der Bewegung der Zunge beteiligt ist,
mindestens eine Zungenbewegung des Patienten zu verfolgen,
dem Patienten basierend auf dem Aktivierungsgrad der identifizierten Gehirnregion und der mindestens einen Zungenbewegung Neurofeedback bereitzustellen und den Patienten anzuweisen, basierend auf dem Neurofeedback mindestens ein motorisches Verhalten der Zunge zu ändern, wodurch die Störung behandelt wird, und
eine Anzeige, die so konfiguriert ist, dass sie dem Patienten Feedback gibt.

2. System gemäß Anspruch 1, das ferner umfasst:
einen MRT-Scanner.

3. System gemäß Anspruch 1, wobei die mindestens eine intakte Gehirnregion aus der Gruppe ausgewählt ist, welche aus Brodmann-Areal 3A, Brodmann-Areal 3B, Brodmann-Areal 2/1, Brodmann-Areal 4, Brodmann-Areal 6, präzentralem Gyrus, dem Kleinhirn, Nucleus dentatus, Culmen, Insula, Basalganglien, Brodmann-Areal 9, inferiorem Frontalgyrus, mittlerem Frontalgyrus, medialem Frontalgyrus und Thalamus besteht.

4. System gemäß Anspruch 1, wobei das MRT-kompatible Zungenmessgerät ein MRT-kompatibler Zungensensor ist, wobei die mindestens eine Zungenbewegung unter Verwendung des MRT-kompatiblen Zungensensors verfolgt wird.

5. System gemäß Anspruch 1, wobei die Steuerung ferner so programmiert ist, dass sie eine erste Sitzung und mindestens eine zweite Sitzung durchführt:
wobei die erste Sitzung umfasst:
Messen einer Größe und einer räumlichen Ausdehnung der Aktivierung des gesamten Gehirns des Patienten während der Bewegung der Zunge durch fMRT, Erzeugen einer Karte eines individualisierten Netzwerks von Gehirnregionen, welche die Bewegung der Zunge steuern, und
Identifizieren mindestens einer intakten Gehirnregion, die funktionell mit einer geschädigten Gehirnregion verbunden ist, basierend auf der Karte des individualisierten Netzwerks von Gehirnregionen, und
wobei die zweite Sitzung umfasst:
weiteres Überwachen des Aktivierungsniveaus in der Vielzahl von Gehirnregionen des Patienten durch funktionelle Echtzeit-Magnetresonanztomographie (fMRT), Verfolgen mindestens einer Zungenbewegung des Patienten,
dem Patienten basierend auf dem Aktivierungsgrad der identifizierten Gehirnregion und der mindestens einen Bewegung der Zunge Neurofeedback bereitzustellen, und
den Patienten anzuweisen, basierend auf dem Neurofeedback mindestens ein motorisches Verhalten der Zunge zu ändern, wodurch die Störung behandelt wird.

6. System gemäß Anspruch 1, wobei der Patient eine Nervenverletzung hat.

7. System gemäß Anspruch 6, wobei die Störung des Schluckens, Kauens oder der Sprachartikulation durch eine Schädigung des Nervus glossopharyngeus oder Nervus hypoglossus verursacht wird.

8. System gemäß Anspruch 1, wobei die Störung des Schluckens, Kauens oder der Sprachartikulation auf neuronaler Ebene entsteht.

9. System gemäß Anspruch 1, wobei die Nervenschädigung auf der supranukleären Ebene liegt.

10. System gemäß Anspruch 1, wobei die Nervenschädigung auf der infranukleären Ebene der Glossopharyngeus- oder Hypoglossusnerven liegt.

11. System gemäß Anspruch 1, wobei das Neurofeedback von dem intakten Bereich des Gehirns, welcher die Zungenbewegung steuert, geliefert wird.

12. System gemäß Anspruch 1, wobei das Lichtwellenleiterkabel (204) umfasst:
ein Beleuchtungsfaserbündel (210), das für eine Verbindung mit der Beleuchtungsquelle (206) konfiguriert ist, und
ein Bildgebungsfaserbündel (212), das für eine Verbindung mit dem Sensor (208) konfiguriert ist.

13. System gemäß Anspruch 12, das ferner eine Beleuchtungsquelle (206), die Licht mit einer Wellenlänge von 400-700 nm emittiert, umfasst.

14. System gemäß Anspruch 1, wobei das Lichtwellenleiterkabel ein Lichtwellenleiterbündel ist, das vier Lichtwellenleiterkabel (204) umfasst, welche jeweils so konfiguriert sind, dass sie eine Beleuchtungsquelle (206) und den Sensor (208) mit dem Mundstück (202) verbinden, wobei ein Ende jedes Lichtwellenleiterkabels (204) der Sensor (208) ist, wobei der Sensor (208) in das Mundstück (202) eingebettet ist und wobei der Lichtwellenleitersensor (200) vier Kanäle umfasst, einen für jedes der Lichtwellenleiterkabel (204).

15. Materielles, nicht transitorisches, computerlesbares Medium, das Anweisungen eines Computerprogramms umfasst, um, wenn sie auf einem Computer zum Steuern des Systems nach Anspruch 1 ausgeführt werden, ein Verfahren zum Behandeln einer Schluck-, Kau- oder Sprachartikulationsstörung, die mit einer geschädigten Gehirnregion bei einem Patienten verbunden ist, durchzuführen, wobei das Verfahren umfasst:
Messen einer Größe und einer räumlichen Ausdehnung der Aktivierung in einer Vielzahl von Gehirnregionen des Patienten während der Bewegung der Zunge durch funktionelle Magnetresonanztomographie (fMRT),
Erzeugen einer Karte eines individualisierten Netzwerks von Gehirnregionen, welche die Zungenbewegung steuern, basierend auf den Messungen des Gehirns des Patienten während der Bewegung der Zunge,
Identifizierung mindestens einer intakten Gehirnregion, die funktionell mit der geschädigten Gehirnregion verbunden ist, basierend auf der Karte des individualisierten Netzwerks von Gehirnregionen, wobei die intakte Region an der Bewegung der Zunge beteiligt ist,
Verfolgung mindestens einer Zungenbewegung des Patienten,
dem Patienten Neurofeedback basierend auf dem Aktivierungsgrad der identifizierten Gehirnregion und der mindestens einen Bewegung der Zunge bereitzustellen, und
den Patienten anzuweisen, mindestens ein motorisches Verhalten der Zunge basierend auf dem vom Blut-Sauerstoff-Niveau abhängigen (BOLD) Signal, welches das Neurofeedback widerspiegelt, zu ändern, wodurch die Störung behandelt wird.

## Revendications

1. Système comprenant :
un dispositif de mesure lingual compatible IRM, comprenant :
un élément buccal, et
un câble à fibre optique configuré pour raccorder une source d'éclairage et un capteur à l'élément buccal ;
un dispositif de commande programmé pour :
mesurer une magnitude et une étendue spatiale d'activation dans une pluralité de régions cérébrales du patient durant un mouvement de la langue, par IRMf ;
générer une carte d'un réseau individualisé des régions cérébrales qui commandent un mouvement de la langue sur la base des mesures du cerveau du patient durant un mouvement de la langue ;
identifier au moins une région cérébrale intacte fonctionnellement associée à une région cérébrale lésée sur la base de la carte du réseau individualisé des régions cérébrales, dans lequel la région intacte est impliquée dans le mouvement de la langue ;
suivre au moins un mouvement de la langue du patient ;
fournir une neuro-rétroaction au patient sur la base du niveau d'activation de la région cérébrale identifiée et du au moins un mouvement de la langue ; et
instruire au patient de changer au moins un comportement moteur de la langue sur la base de la neuro-rétroaction, traitant ainsi le trouble ; et
un affichage configuré pour fournir une rétroaction au patient.

2. Système selon la revendication 1, comprenant en outre :
un scanner IRM.

3. Système selon la revendication 1, dans lequel la au moins une région cérébrale intacte est sélectionnée dans le groupe constitué de l'aire de Brodmann 3A, aire de Brodmann 3B, aire de Brodmann 2/1, aire de Brodmann 4, aire de Brodmann 6, d'une circonvolution précentrale, du cervelet, du noyau dentelé, du culmen, du cortex insulaire, des noyaux gris centraux, de l'aire de Brodmann 9, de la circonvolution frontale inférieure, circonvolution frontale moyenne, circonvolution frontale médiane et du thalamus.

4. Système selon la revendication 1, dans lequel le dispositif de mesure lingual compatible IRM est un capteur lingual compatible IRM, dans lequel le au moins un mouvement de la langue est suivi en utilisant le capteur lingual compatible IRM.

5. Système selon la revendication 1, dans lequel le dispositif de commande est en outre programmé pour mettre en œuvre une première session et au moins une seconde session :
dans lequel la première session comprend les étapes consistant à :
mesurer une magnitude et une étendue spatiale d'activation du cerveau entier du patient durant un mouvement de la langue par IRMf ;
générer une carte d'un réseau individualisé des régions cérébrales qui commandent un mouvement de la langue ; et
identifier au moins une région cérébrale intacte fonctionnellement associée à une région cérébrale lésée sur la base de la carte du réseau individualisé des régions cérébrales ; et
dans lequel la seconde session comprend les étapes consistant à :
surveiller de manière supplémentaire le niveau d'activation dans la pluralité de régions cérébrales du patient par imagerie par résonance magnétique fonctionnelle (IRMf) en temps réel ;
suivre au moins un mouvement de la langue du patient ;
fournir une neuro-rétroaction au patient sur la base du niveau d'activation de la région cérébrale identifiée et du au moins un mouvement de la langue ; et
instruire au patient de changer au moins un comportement moteur de la langue sur la base de la neuro-rétroaction, traitant ainsi le trouble.

6. Système selon la revendication 1, dans lequel le patient présente une lésion d'un nerf.

7. Système selon la revendication 6, dans lequel le trouble de déglutition, mastication, ou d'articulation de la parole est causé par une détérioration du nerf glossopharyngé ou hypoglosse.

8. Système selon la revendication 1, dans lequel le trouble de déglutition, mastication, ou d'articulation de la parole provient du niveau neuronal.

9. Système selon la revendication 1, dans lequel la détérioration neurologique a lieu au niveau supranucléaire.

10. Système selon la revendication 1, dans lequel la détérioration neurologique a lieu au niveau infranucléaire des nerfs glossopharyngé ou hypoglosse.

11. Système selon la revendication 1, dans lequel la neuro-rétroaction est délivrée depuis la région intacte du cerveau qui commande un mouvement de la langue.

12. Système selon la revendication 1, dans lequel le câble à fibre optique (204) comprend :
un faisceau de fibres d'éclairage (210) configuré pour le raccordement à la source d'éclairage (206) ; et
un faisceau de fibres d'imagerie (212) configuré pour le raccordement au capteur (208).

13. Système selon la revendication 12, comprenant en outre une source d'éclairage (206) qui émet de la lumière avec une longueur d'onde de 400 à 700 nm.

14. Système selon la revendication 1, dans lequel le câble à fibre optique est un faisceau de fibres optiques comprenant quatre câbles à fibre optique (204), chacun configuré pour raccorder une source d'éclairage (206) et le capteur (208) à l'élément buccal (202), dans lequel une extrémité de chaque câble à fibre optique (204) est le capteur (208), ledit capteur (208) est enchâssé dans l'élément buccal (202), et dans lequel le capteur à fibre optique (200) comprend quatre canaux, un pour chacun des câbles à fibre optique (204).

15. Support lisible par ordinateur, non transitoire, tangible, comprenant des instructions de programme informatique pour mettre en œuvre, lorsqu'exécutées sur un ordinateur pour commander le système selon la revendication 1, un procédé de traitement d'un trouble de déglutition, mastication, ou d'articulation de la parole associé à une région cérébrale lésée chez un patient, le procédé comprenant les étapes consistant à :
mesurer une magnitude et une étendue spatiale d'activation dans une pluralité de régions cérébrales du patient durant un mouvement de la langue, par imagerie par résonance magnétique fonctionnelle (IRMF) ;
générer une carte d'un réseau individualisé des régions cérébrales qui commandent un mouvement de la langue sur la base des mesures du cerveau du patient durant un mouvement de la langue ;
identifier au moins une région cérébrale intacte fonctionnellement associée à la région cérébrale lésée sur la base de la carte du réseau individualisé des régions cérébrales, dans lequel la région intacte est impliquée dans le mouvement de la langue ;
suivre au moins un mouvement de la langue du patient ;
fournir une neuro-rétroaction au patient sur la base du niveau d'activation de la région cérébrale identifiée et du au moins un mouvement de la langue ; et
instruire au patient de changer au moins un comportement moteur de la langue sur la base du signal dépendant du niveau d'oxygène sanguin (BOLD) qui reflète la neuro-rétroaction, traitant ainsi le trouble.
